# EUROPÄISCHE PATENTSCHRIFT

(11) **EP 1 131 422 B1**
(45) Veröffentlichungstag und Bekanntmachung des Hinweises auf die Patenterteilung: **31.10.2007**
(21) Anmeldenummer: 99958044.2
(22) Anmeldetag: 16.11.1999
(51) Int. Cl.: C12N 15/11, C07K 14/47, C07K 14/505, A61K 38/04

(54) **REKOMBINANTE GLYCOPROTEINE, VERFAHREN ZU IHRER HERSTELLUNG, SIE ENTHALTENDE ARZNEIMITTEL UND IHRE VERWENDUNG**
RECOMBINANT GLYCOPROTEINS, METHOD FOR THE PRODUCTION THEREOF, MEDICAMENTS CONTAINING SAID GLYCOPROTEINS AND USE THEREOF
GLYCOPROTEINES RECOMBINANTES, LEUR TECHNIQUE DE PRODUCTION, MEDICAMENTS LES CONTENANT ET LEUR UTILISATION

(30) Priorität: 16.11.1998 DE 19852729
(43) Veröffentlichungstag der Anmeldung: 12.09.2001
(73) Patentinhaber: Reutter, Werner, Prof. Dr., D-14195 Berlin (DE)
(72) Erfinder: REUTTER, Werner, D-14195 Berlin (DE); TAUBER, Rudolf, D-14163 Berlin (DE); HORSTKORTE, Rüdiger, D-13156 Berlin (DE); NÖHRING, Sabine, D-14476 Seeburg (DE); GOHLKE, Martin, D-14199 Berlin (DE); NUCK, Rolf, D-12307 Berlin (DE); SCHMIDT, Richard, R., D-78465 Konstanz (DE); HAUSER, Charlotte, D-80639 München (DE)
(74) Vertreter: Helbing, Jörg
(86) Internationale Anmeldenummer: PCT/EP1999/008787
(87) Internationale Veröffentlichungsnummer: WO 2000/029567

(56) Entgegenhaltungen:
- WO-A-94/24167
- CHEMICAL ABSTRACTS, vol. 126, no. 19, 12. Mai 1997 (1997-05-12) Columbus, Ohio, US; abstract no. 246554, M GOHLKE ET AL.: "Analysis of site-specific N-glycosylation of recombinant Desmodus rotundus salivary plasminogen activator rDSPA.alph.1 expressed in Chinese hamster ovary cells" XP002134014 & GLYCOBIOLOGY, Bd. 7, Nr. 1, 1997, Seiten 67-77,

## Beschreibung

Die Erfindung betrifft neue rekombinante Glycoproteine, die als Botenstoffe, Signalstoffe, Promotoren, Stimulatoren und Initiatoren in vielfältiger Weise in den tierischen, insbesondere menschlichen Kreislauf eingreifen; Verfahren zu ihrer Herstellung, sie enthaltende pharmazeutische Mittel und ihre Verwendungen. Die Erfindung betrifft insbesondere neue rekombinante humane Glycoproteine (rh Glycoproteine), vorzugsweise neue rh Differenzierungsfaktoren, insbesondere rh Erythropoietin; neue rh Wachstumsfaktoren, insbesondere rh CSF (Colony-Stimulating-Factor), rh GMCSF (Granulocyten-Monocyten-Stimulationsfaktor); neue rh Thrombolytika, insbesondere rh tPA (Tissue-Plasminogen-Activator) und rh Urokinase; neue rh Thromboprophylaktika, insbesondere rh Antithrombin III; neue rh Gerinnungsfaktoren, insbesondere rh den Faktor VIII und rh Faktor IX; neue rh Interferone, insbesondere rh α-, β- und γ-Interferone; und neue rh Interleukine, insbesondere rh IL-2, IL-15, IL-16 und IL-17; Verfahren zu ihrer Herstellung; sie enthaltende pharmazeutische Mittel und ihre Verwendungen.

Als Glycoproteine werden Proteine bezeichnet, die kovalent gebundene Kohlenhydrate (Zucker) enthalten. Glycoproteine, insbesondere Human-Glycoproteine, wirken als Botenstoffe, Signalstoffe, Promotoren, Initiatoren oder Stimulatoren von Stoffwechselprozessen bei Tieren und Menschen, die direkt oder indirekt in diese Stoffwechselprozesse eingreifen. Sie werden üblicherweise im Körper in vivo oder biosynthetisch gebildet und, nachdem sie ihre spezifische Funktion erfüllt haben, von diesem wieder ausgeschieden oder abgebaut. Die im Körper in natürlicher Weise gebildeten Glycoproteine (sogenannte "native Glycoproteine") sind dadurch charakterisiert, daß sie an der Aminogruppe des Aminoglycan-Restes durch eine Acetylgruppe substituiert sind. Diese nativen Glycoproteine, die auf natürlichem Wege in vivo oder auf biosynthetischem Wege hergestellt werden können (vgl. DE-PS 4 311 580), stellen bei Mensch und Tier wirksame Mittel dar zur Stimulierung des Wachstums und der Differenzierung von menschlichen und tierischen Zellen des Immunsystems und zur Verhinderung der Adhäsion von Leukozyten, Thrombozyten und Tumorzellen an Gefäßendothelzellen; zur Stimulierung des Immunsystems, insbesondere von T-Lymphozyten, zur Infektabwehr, zur Behandlung von Immunschwächen, Tumorerkrankungen einschließlich Metastasierungsprozessen, Infektionserkrankungen und Kreislaufversagen, insbesondere Gefäßvarschlüssen und Septikämien; zur Hemmung der Bindung eines Liganden an seinen sialylierten Zelloberflächen-Rezeptor; zur Hemmung der Bindung eines mikrobiellen Phagen oder Toxins an die Wirtszelle über einen sialylierten Rezeptor durch in vivo-Modulation von Neuraminsäuren; zur biosynthetischen Herstellung von Liganden oder Rezeptoren mit modifizierter Neuraminsäure und Verwendung derselben zur Kompetition physiologischer oder pathologischer Ligand-Rezeptor-Interaktionen; zur in vitro-Beeinflussung des Infektionsverlaufs mit humanen Immundeffizienz-viren sowie zur in vivo-Prävention der Infektion mit humanen Immundeffizienz-Viren; und zur Behandlung von parasitären Erkrankungen.

In tierischen Organismen kommen Glycoproteine als wesentliche Bestandteile von Zellmembranen sowie als lösliche Komponenten von Körperflüssigkeiten und der extrazellulären Matrix vor. Die Kohlenhydrate sind zu Ketten verknüpft (Oligosaccharide) und können auf unterschiedliche Weise mit dem Proteingerüst verknüpft sein. Sie enthalten als wichtige Bestandteile der Zellmembran Sialinsäure (Derivate der 2-Keto-3-desoxy-D-glycero-D-galacto-nonulopyranosidonsäure (KDN)), der eine bedeutende Rolle bei biologischen Prozessen zukommt.

Je nach ihrem Bindungstyp werden die Oligosaccharide unterschiedlichen Gruppen zugeordnet. Die Oligosaccharide von säurethioglycoproteinen sind am häufigsten N-glycosidisch an einen Asparaginrest der Polypeptidkette gebunden (N-Glycane). In dieser Gruppe finden sich einerseits sezernierte Glycoproteine mit unterschiedlichen Funktionen, z.B. lösliche Enzyme, Immunglobuline und Hormone, andererseits Membranglycoproteine, z.B. Membranenzyme, Transportproteine sowie Rezeptorproteine. Eine weitere Gruppe bilden die O-glycosidisch über einen Galatose-, N-Acetylgalactosamin- oder Xyloserest an einen Serin- oder Threoninrest der Polypeptidkette gebundenen Oligosaccharide (O-Glycane). Zusammen mit N-Glycanen kommen sie auch in Immunglobulinen und anderen Glycoproteinen vor. Zu den O-Glycanen gehören auch die oligosaccharide der Proteoglycane, die sich durch einen besonders hohen Kohlenhydratanteil auszeichnen. In diesen in der extrazellulären Matrix vorkommenden Glycokonjugaten können die oligosaccharide über einen Galactose-, N-Acetylgalactosamin- oder Xyloserest an das Polypeptidgerüst gebunden sein.

Die Glycoproteine, bestehend aus Monosacchariden und Eiweiß, werden häufig mit den Glycolipiden als Glycokonjugate zusammengefaßt. Die Zucker-Komponenten der Glycoproteine, die mit wenigen Ausnahme weniger als 50 % des Gesamt-Glycoproteins ausmachen, sind über durch Glycosidasen spaltbare O- oder N-glycosidische Bindungen mit dem Peptidanteil verknüpft. Als Kohlenhydrate finden sich in den Glycoproteinen Hexosen (Galactose, Mannose, seltener Glucose), N-Acetylhexosamine, N-Acetylneuraninsäure, Fucose und andere. Zur Identifizierung und Bestimmung der Glycoproteine eignet sich in erster Linie die Affinitätschromatographie mit pflanzlichen Lectinen als Liganden (z.B. Concanavalin A oder Weizenkeimagglutinin oder andere).

Zu den Glycoproteinen gehören fast alle Membran-Glycoproteine, Serumproteine, Plasmaproteine, die Blutgruppensubstanzen, viele Enzyme und Proteohormone, alle Antikörper, die Chalone, Muzine, Lectine, Bindine, Fibronectin, der Intrinsic-Faktor und dgl. Als Membran- oder oberflächen-Proteine spielen manche Glycoproteine für die Pathogenität von Viren eine Rolle. Hier wie bei anderen Rezeptors-pezifischen zellulären Wechselwirkungen sind die Kohlenhydrat-Komponenten für Erkennungsprozesse auf molekularer Ebene verantwortlich. Über bestimmte Zuckerstrukturen auf Rezeptoren heften einige Bakterien und Viren an ihre Zielzellen an.

Besonders bedeutungsvoll sind Oligosaccharid-Strukturen auch im Hinblick auf die Zell-Zell- bzw. Zell-Matrix-Interaktion. So vermitteln die Oligosaccharide von Glycoproteinen die Adhäsion von Neuralzellen und die Bindung von Lymphozyten an spezifische Endothelzellen. Außerdem können Oligosaccharide als antigene Determinanten von Glycoproteinen dienen. Auch während der Embryogenese und der Organogenese sind Kohlenhydrat-Kohlenhydrat-Wechselwirkungen wesentlich an der spezifischen Zellerkennung beteiligt. Die maligne Transformation von Zellen ist von charakteristischen Veränderungen der Oligosaccharid-Strukturen von Glycoproteinen begleitet. Inwieweit veränderte Oligosaccharid-Strukturen von Tumorzellen und Zell-Glycoproteinen dabei Ursache oder Wirkung der Tumorentstehung und Metastasierung sind, ist bis jetzt nicht eindeutig geklärt.

In Krankheitsfällen, in denen das Immunsystem beteiligt ist, ist die Unterstützung des Immunsystems durch Verabreichung von Substanzen, die Zellen des Immunsystems stimulieren, erforderlich. Die suche nach wirkstoffen zur Stimulierung des Immunsystems ist daher ein bevorzugtes Ziel pharmakologischer Forschung. Wirkungsvolle Immunstimulantien mit wenig oder keinen Nebenwirkungen sind aber bisher nicht bekannt.

Aufgabe der Erfindung war es daher, neue Substanzen zu finden, mit deren Hilfe es möglich ist, die von Glycoproteinen als Botenstoffe, Signalstoffe, Promotoren, stimulatoren und Initiatoren gesteuerten Stoffwechselprozesse noch wirksamer, spezifischer und selektiver als bisher zu beeinflussen. Aufgabe der Erfindung war es insbesondere solche neuen Glycoproteinen als Botenstoffe, Signalstoffe, Stimulatoren, Promotoren und Initiatoren für den menschlichen und tierischen Stoffwechsel zu finden, die in geringerer Dosis eingesetzt werden können und daher weniger unerwünschte Nebenwirkungen haben als die entsprechenden nativen Stoffe.

Es wurde nun gefunden, daß diese Aufgabe erfindungsgemäß dadurch gelöst werden kann, daß bei den obengenannten nativen Glycoproteinen die natürlicherweise vorhandene Acetylsubstitution in dem Aminoglycan-Rest durch eine andere Acylgruppe, insbesondere durch eine (C₃-C₇)-Acylgruppe, oder einen Hydrocarbylrest, insbesondere einen (C₃-C₇)-Alkyl-, -Alkenyl- oder -Alkinylrest, ersetzt ist- Es hat sich nämlich gezeigt, daß die dabei erhaltenen neuen Verbindungen eine veränderte, insbesondere verlängerte, Pharmakokinetik, verglichen mit der biologischen Halblebenszeit (Halbwertszeit) der entsprechenden bekannten und handelsüblichen nativen Glycoproteine mit einer N-Acetylgruppe im Aminoglycanrest, aufweisen. Diese neuen Glycoproteine lassen sich auf die weiter unten beschriebene Weise insbesondere auf gentechnischem Wege herstellen, wobei man rekombinante Glycoproteine, insbesondere rekombinante Human-Glycoproteine (rh Glycoproteine), erhält.

Aufgrund ihrer veränderten, insbesondere verlängerten, Pharmakomkinetik können diese Substanzen in wesentlich geringeren Dosen therapeutisch eingesetzt werden, wodurch auch die bisher bei Verwendung der entsprechenden nativen Substanzen festzustellenden unerwünschten Nebenwirkungen beträchtlich herabgesetzt werden können. Dies gilt insbesondere dann, wenn die neuen Glycoproteine in Form ihrer Derivate eingesetzt werden, in denen die OH-Gruppen des Monosaccharid-Anteils einfach oder mehrfach acyliert, vorzugsweise acetyliert, ist (sind).

Gegenstand der vorliegenden Erfindung sind gemäß einem ersten Aspekt neue rekombinante Glycoproteine, insbesondere rekombinane Human-Glycoproteine (rh Glycoproteine) der allgemeinen Formel (I) 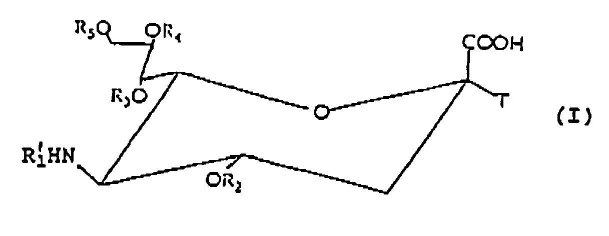 worin bedeuten:
R'₁ einen cyclischen (C₃-C₇)-Acylrest, der eine oder mehrere Hydroxyl- und/oder Carbonylgruppen aufweisen kann,
R₂, R₃, R₄ und R₅, die gleich oder verschieden sein können, jeweils Wasserstoff, einen linearen oder verzweigten C₁₋₂₀-Alkylrest; einen linearen oder verzweigten C₃-₂₀-Alkenylrest; einen linearen oder verzweigten C₃₋₂₀-Alkinylrest; einen C₄₋₂₀-Alkenyl- oder Alkinylrest mit 2 oder mehr Doppelbindungen bzw. Dreifachbindungen; einen C₆₋₂₀-Arylrest; einen linearen oder verzweigten, gesättigten oder ein- oder mehrfach ungesättigten Acylrest (-CO-R₁) mit insgesamt 1 bis 20 Kohlenstoffatomen, der eine oder mehrere Hydroxylgruppen aufweisen kann; einen C₆₋₂₀-Aroylrest; einen Carbonylamidrest der Formel -CONH₂ oder - CONHR₁; einen linearen oder verzweigten, gesättigten oder ein- oder mehrfach ungesättigten Thioacylrest (-CS-R₁) mit insgesamt 1 bis 20 Kohlenstoffatomen; oder einen Thiacarbamidrest der Formel -CS-NH₂ oder -CS-NHR₁; wobei R₁ jeweils für Wasserstoff oder einen linearen oder verzweigten, gesättigten oder ein- oder mehrfach ungesättigten C₁₋₂₀-Alkylrest steht und wobei jeder der vorgenannten Reste außer H eine oder mehrere Halogen-, Hydroxy-, Epoxy-, Amino-, Mercaptan-, Phenyl-, Phenol- oder Benzylgruppen als Substituent(en) aufweisen kann und
T einen Mono-, Di- oder Oligosaccharidrest mit bis zu 40 glykosidisch miteinander verknüpften Zuckerresten, die Furanose- und/oder Pyranoseringe darstellen, und 5 bis 230 Kohlenstoffatome enthalten und N- oder 0-glycosidisch an Polypeptide gebunden sind.

Vorzugsweise bedeuten in der obengenannten allgemeinen Formel (I):
R₂, R₃, R₄ und R₅, die gleich oder verschieden sein können, jeweils Wasserstoff, einen linearen oder verzweigten Alkylrest mit 1 bis 7 Kohlenstoffatomen; einen linearen oder verzweigten Alkenylrest mit 3 bis 10 Kohlenstoffatomen; einen linearen oder verzweigten Alkinylrest mit 3 bis 10 Kohlenstoffatomen; einen Alkenyl- bzw. Alkinylrest mit 2 oder mehr Doppelbindungen bzw. Dreifachbindungen mit 7 bis 12 Kohlenstoffatomen; einen Phenylrest; einen linearen oder verzweigten, gesättigten oder ein- oder mehrfach ungesättigten Acylrest (-CO-R₁) mit insgesamt 1 bis 7 Kohlenstoffatomen, insbesondere Acetylrest, einschließlich seiner ein- oder mehrfach hydroxylierten Analoga; einen Aroylrest mit 6 bis 10 Kohlenstoffatomen: einen carbonylamidrest der Formel -CONH₂ oder - CONHR₁; einen linearen oder verzweigten, gesättigten oder ein- oder mehrfach ungesättigten Thioacylrest (-CS-R₁) mit insgesamt 1 bis 7 Kohlenstoffatomen; oder einen Thiocarbamidrest der Formel -CS-NH₂ oder -CS-NHR₁; wobei R₁ jeweils für Wasserstoff oder einen linearen oder verzweigten, gesättigten oder ein- oder mehrfach ungesättigten Alkylrest mit 1 bis 20 Kohlenstoffatomen steht und jeder der vorgenannten Reste außer H gegebenenfalls ein- oder mehrfach substituiert sein kann durch Fluor, Chlor, Brom oder Jod, Hydroxy-, Epoxy-, Amino-, Mercaptan-, Phenyl-, Phenol- oder Benzylgruppen.

Gemäß einer weiteren bevorzugten Ausführungsform der Erfindung steht in der allgemeinen Formel (I) T
für einen Saccharidrest mit N-Glycan-struktur der Formel (Ia) 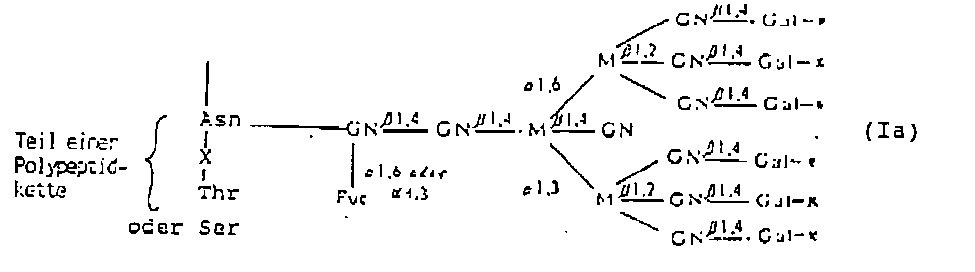 worin bedeuten:
- Gal =: Galactose,
- GN =: N-Acetyl-D-glucosamin,
- M =: D-Mannose,
- Fuc =: Fucose,
- Asn =: Asparagin,
- X =: eine beliebige Aminosäure außer Prolin,
- Thr =: Threonin,
- Ser =: Serin,
- * =: T-Verknüpfungsstelle (1 bis 6 Molekülreste),
wobei beide peripheren Reste M durch 1 bis 3 Trisaccharide substituiert sein können; oder
für einen Saccharidrest mit O-Glycan-Struktur der allgemeinen Formel (Ib): 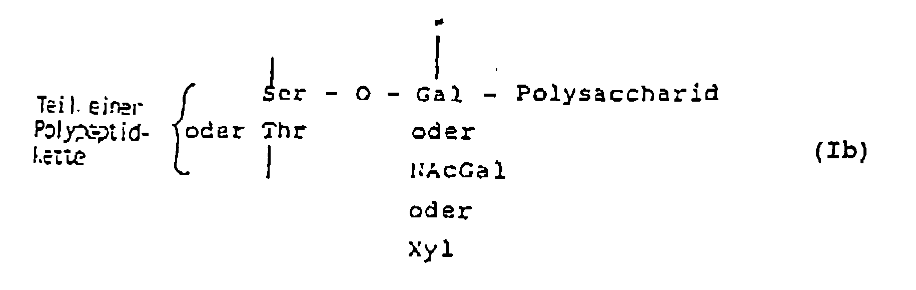 worin bedeuten:
- Gal =: Galactose
- Thr =: Threonin
- Ser =: serin
- Xyl =: Xylose
- NAcGal =: N-Acetyl-galactosamin
- * =: T-Verknüpfungsstelle,
wobei in den obigen Formeln (Ia) und (Ib) die Galactose (Gal) ersetzt sein kann durch 2-Desoxy-galactose oder 2-Desoxy-2-halogenid(F, Cl, Br, J)-galactose.

Vorzugsweise steht insbesondere dann, wenn T einen Saccharidrest mit N-Glycanstruktur oder einen Saccharidrest mit O-Glycanstruktur darstellt, GN für einen Rest der allgemeinen Formel (II): 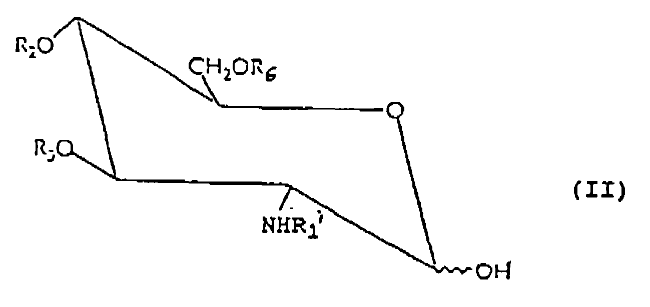 in der R'₁, R₂ und R₃ die vorstehend angegebenen Bedeutungen haben, R₆ die gleichen Bedeutungen wie R₂ und R₃ hat und -NHR'₁ neben der äquatorialen Position auch die axiale Position einnehmen kann, und wobei außerdem bei äquatorialer Position von -NHR'₁ -OR₂ in axialer Position stehen kann, und in der eine oder mehrere oder alle der Gruppen -OR₂, -OR₃, -OR₆ und/oder die freie OH-Gruppe acyliert sein können durch einen linearen oder verzweigten, gesättigten oder ein- oder mehrfach ungesättigten Acylrest (-CO-R₁) mit insgesamt 1 bis 20, vorzugsweise 1 bis 7 Kohlenstoffatomen, vorzugsweise durch den Acetylrest (-CO-CH₃), einschließlich seiner ein- oder mehrfach hydroxylierten Analoga.

Die oben angegebenen Reste R₁ bis R₆ stehen vorzugsweise für H oder CH₃ oder (C₁-C₇)-Acyl, insbesondere Acetyl.

In der HHR'₁-Gruppe steht R'₁ vorzugsweise für einen Cyclopropanoylrest, der eine oder mehrere Hydroxyl- und/oder Carbonylgruppen aufweisen kann.

Die vorstehend angegebenen neuen erfindungsgemäßen Verbindungen weisen eine veränderte, insbesondere verlängerte, Pharmakokinetik auf, verglichen mit der biologischen Halblebenszeit (Halbwertszeit) der entsprechenden bekannten und handelsüblichen natürlichen (nativen) Glycoproteine mit R'₁ = Acetyl.

Bei den erfindungsgemäßen neuen Verbindungen handelt es sich vorzugsweise um
rekombinante Glycoproteine, speziell um rekombinante Human-Glycoproteine (rh Glycoproteine), vorzugsweise um rh Differenzierungsfaktoren, insbesondere rh Erythropoietin;
um rh Wachstumsfaktoren, insbesondere rh CSF (Colony-Stimulating-Factor), rh GMCSF (Granulocyten-Monocyten-Stimulationsfaktor);
um rh Thrombolytika, insbesondere rh Tissue-Plasminogen-Activator (tPA) und rh Urokinase;
um rh Thromboprophylaktika, insbesondere rh Antithrombin III;
um rh Gerinnungsfaktoren, insbesondere den rh Faktor VIII und rh Faktor IX;
um rh Interferone, insbesondere rh α-, β- und γ-Interferone; und
um rh Interleukine, insbesondere rh IL-2, IL-15, IL-16 und IL-17.

Die vorstehende definierten erfindungsgemäßen rekombinanten Glycoproteine, insbesondere rh Glycoproteine, stellen neue Substanzklassen dar, die als potente Wirkstoffe zur Behandlung von Krankheiten eingesetzt werden können, an denen Zellen der spezifischen und unspezifischen Immunabwehr, Tumorzellen, Leukozyten, Thrombozyten und Gefäßendothelzellen beteiligt sind. Die erfindungsgemäßen neuen Verbindungen können gezielt über eine Modulation Membran-ständiger Rezeptoren wirken, die an der Regulation des Wachstums und der Differenzierung sowie der Adhäsion von Zellen des Immunsystems, von Tumoren und von Gefäßen beteiligt sind. Eine Immunstimulation ist notwendig bei Heilungsprozessen infektiöser und tumorbedingter Erkrankungen. Außerdem verhindern die erfindungsgemäßen neuen Verbindungen die Adhäsion von Leukozyten oder Tumorzellen auf Gefäßendothelzellen bei septischem Schock oder bei der Metastasierung. Die Verabreichung dieser Substanzen, kann aufgrund ihrer veränderten, vorzugsweise verlängerten, biologischen Halbwertszeit in einer deutlich verminderten Dosierung erfolgen. Bei Verwendung ihrer an den OH-Gruppen des Monosaccharidrestes acylierten, insbesondere acetylierten, Derivate kann die Dosierung bis auf etwa die Hälfte weiter herabgesetzt werden kann. Die erfindungsgemäßen neuen rekombinanten Glycoproteine können als Stimulantien für Zelln des Immunsystems eingesetzt werden. Dadurch ist eine beträchtliche Stärkung des Immunsystems bei immungeschwächten Organismen möglich. Sie zeichnen sich durch eine hohe Zell-Spezifität und geringe bis vollständig fehlende Nebenwirkungen, sowie eine hohe Stabilität, insbesondere biologische Stabilität, aus.

Die Erfindung wird nachstehend unter Bezugnahme auf das spezifische Glycoprotein rh Erythropoietin näher erläutert, ohne jedoch darauf beschränkt zu sein. Selbstverständlich gelten die nachstehenden Ausführungen, wie für den Fachmann ohne weiteres ersichtlich, auch für die anderen, oben aufgezählten neuen rekombinanten Glycoproteine der Erfindung.

Humanes Erythropoietin (EPO) ist ein Glycoprotein mit 166 Aminosäuren, 3 Glycosylierungsstellen an den Aminosäure-Positionen 34, 38 und 83 und einem Molekulargewicht von etwa 34 000 bis 38 000. Der Anteil der Glycosyl-Seitenketten am Molekulargewicht beträgt etwa 40 % (vgl. Jacobs et al, "Nature" 313, 806-809 (1985); und Dordal et al, "Endocrinology" 116, 2293-2299 (1985)). EPO wird in der Niere gebildet und zu seinem physiologischen Wirkort, dem Knochenmark, über den Blutweg transportiert.

EPO läßt sich entweder aus natürlichen Quellen, wie menschlichem Urin, isolieren (vgl. z.B. Miyake et al, "J. Biol. Chem. ", 252, 5558-5564 (1977)), oder es kann auf gentechnischem Wege hergestellt werden (vgl. z.B. EP-A-0 148 605 und 0 267 678). Seine physiologische Funktion besteht in der Stimulation der Erythropoese, d.h. es stimuliert Wachstum und Differenzierung von Erythrozyten-Vorläuferzellen. Es wird in großem Umfang als Heilmittel für Anämie-Patienten, für postoperative Patienten und Patienten, die nach einer Nierenexstirpation eine Homo-Dialyse erhalten, angewendet. In erster Linie dient es der Therapie renal bedingter Anämien, d.h. für Anämien, die durch die Einschränkung oder den Verlust der Funktion der Nieren entstanden sind. Der Funktionsverlust der Nieren kann verschiedene Ursachen haben, beispielsweise Minderdurchblutung, chronische Entzündungen, Traumata, Exstirpation, hepatorhenales Syndrom und durch diese Erkrankungen bedingte Dialysemaßnahmen.

Es ist bereits ein Verfahren zum Isolieren von EPO aus menschlichem Urin, der EPO enthält, bekannt (vgl. z.B. US-Patent 3 865 801). Da jedoch der Gehalt an EPO in üblichem menschlichem Urin extrem niedrig ist und in der Größenordnung von etwa 0,01 bis 0,02 Gew.-% des gesamten Proteins in dem Urin liegt, ist es schwierig, EPO auf diesem Wege effektiv herzustellen. Es ist auch bereits ein Verfahren aus EP 0 116 446 bekannt, nach dem hochreines Erythropoietin hergestellt werden kann und in dem ein Polypeptid als Antikörper verwendet wird. Diese Druckschrift enthält jedoch keine Angaben über die dabei erzielbaren Ausbeuten und dieses Verfahren ist verhältnismäßig schwierig durchführbar.

Seit einigen Jahren ist das Gen für menschliches EPO bekannt, das aus einer foetalen Leber-Genbank isoliert und charakterisiert werden konnte. Es steht seit 1985 für gentechnologische Untersuchungen zur Verfügung (vgl. Jacobs et al, "Nature" 313, 806-809 (1985)). EPO kann mit Hilfe gentechnologischer Verfahren in tierischen Zellen exprimiert werden.

Das erfindungsgemäße neue rekombinante EPO kann (ebenso wie die übrigen obengenannten neuen rekombinanten Glycoproteine) auf gentechnischem Wege hergestellt werden, indem man von reinem Erythropoietin aus einem Erythropoietin enthaltenden Material ausgeht, das aus Zellkulturüberständen gewonnen wurde. Als Kulturzellen werden eucaryote, vor allem tierische Zellen, insbesondere nicht-transformierte und transformierte CHO-Zellen oder COS7-Zellen oder Nalm-Zellen oder Fibroblaste eingesetzt, die in einem üblichen Kulturmedium, vorzugsweise in einem FCS-, RPMI 1640-, HAT-, Eagle-MEM-, Dulbecco-MEM- und/oder Glasgow-MEM-Medium, das als Glycanvorläufer ein cyclisches N-(C₃-C₇)-acyliertes Monosaccharid für die Biosynthese enthält, in an sich bekannter Weise gezüchtet und das dabei erhaltene rh EPO gegebenenfalls auf ebenfalls an sich bekannte Weise (vgl. "Organicum") an den OH-Gruppen des Monosaccharid-Restes vorzugsweise mittels eines geeigneten Säureanhydrids, insbesondere mittels Essigsäureanhydrid, ein- oder mehrfach oder vollständig acyliert bzw. vorzugsweise acetyliert wird.

Gegenstand der vorliegenden Erfindung ist gemäß einem weiteren Aspekt ein Verfahren zur Herstellung der weiter oben definierten neuen rekombinanten Glycoproteine der Erfindung aus einem ein oder mehrere rekombinante Glycoproteine, insbesondere rekombinante Human-Glycoproteine, enthaltenden Material, das aus Zellkulturüberständen gewonnen wurde, das dadurch gekennzeichnet ist, daß man als Kulturzellen eukaryote, vorzugsweise tierische Zellen, besonders bevorzugt nicht-transformierte oder transformierte CHO-Zellen oder COS7-Zellen oder Nalm-Zellen oder Fibroblaste, in einem üblichen Kulturmedium, das als Glycanvorläufer ein cyclisches N-(C₃-C₇)-acyliertes Monosaccharid für die Biosynthese der Aminozucker in einer Konzentration von 0,001 bis 50 mMol/1, vorzugsweise von 0,5 bis 20 mMol/l, enthält, bei einer Temperatur von 18 bis 40°C, vorzugsweise von 30°C, und bei einem pH-Wert von 6,0 bis 8,2, vorzugsweise von 7,2, in an sich bekannter Weise kultiviert und das dabei erhaltene N-Acylderivat bzw. N-Hydrocarbylderivat des Glycoproteins in an sich bekannter Weise abtrennt und gewinnt und gegebenenfalls auf an sich bekannte Weise an den OH- bzw. OR-Gruppen des Monosaccharids vorzugsweise mittels eines geeigneten Säureanhydrids, insbesondere mittels Essigsäureanhydrid, ein- oder mehrfach oder vollständig acyliert bzw. vorzugsweise acetyliert.

Als Kulturmedium wird vorzugsweise ein FCS-, RPMI 1640-, HAT-, Eagle-MEM-, Dulbecco-MEM- und/oder Glasgow-MEM-Medium verwendet.

Als Glycanvorläufer verwendet man cyclische N-(C₃-C₇)-Acyl- insbesondere N-Cyclopropanoyl-, D-glucosamin, -D-galactosamin oder -neuraminsäure, insbesondere -D-mannosamin, oder eine Mischung davon.

Eines der wesentlichen Merkmale des erfindungsgemäßen Verfahrens besteht darin, daß ein Zellkulturmedium verwendet wird, das als Glycanvorläufer ein entsprechend N-acryliertes Manosacaharid für die Biosynthese der Aminozucker enthält, beispielsweise N-cyclopropanoyl-D-glucosamin oder N-cyclopropanoyl-D-galactosamin, die dazu geeignet sind, N-Acetyl-D-glucosamin oder M-Acetyl-D-galactosamin oder N-Acetyl-neuraminsäure durch N-cyclopropanoyl-D-glucosamin oder N-cyclopropanoyl-D-galactosamin oder N-cyclopropanoyl-neuraminsäure in der Glycankette des aus den Kulturzellen sezernierten EPO zu ersetzen. Als besonders wirkungsvoll haben sich direkte Vorläufer der Neuraminsäure erwiesen, nämlich N-Cyclopropanoyl-D-mannosamin oder ein Gemisch aus diesen Zukkern. Der Stoffwechselweg dieser Aminozuckeranaloga ist aufgeklärt (vgl. Kayser et al, "J. Biol. Chem." 267, 16934-16938 (1992)), und nachstehend für N-Propanoyl-D-mannosamin wiedergegeben: 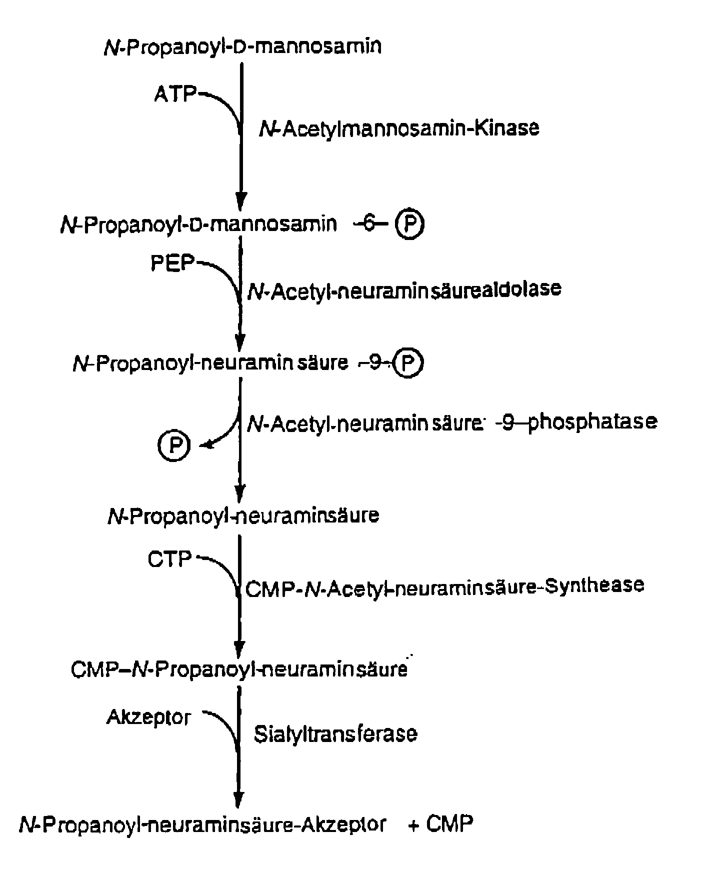 wobei in der obengenannten Reaktionsfolge bedeuten:
- ATP: Adenosintriphosphat
- PEP: Phosphoenolpyruvat
- P: Phosphat
- CTP: Cytidintriphosphat
- CMP: Cytidinmonophosphat
- Akezptor: ein Protein, das sich im Zustand der Glycosylierung befindet.

Für die Glycosylierung von Proteinen werden die folgenden Monosaccharide benötigt: N-Acetyl-D-glucosamin, N-Acatyl-D-galactosamin, D-Mannose, D-Galactose, L-Fucose, N-Acetyl-D-mannosamin und N-Acetyl-neuraminsäure, die in typischer Weise miteinander verknüpft sind (vgl. E. Buddekke, "Grundriß der Biochemie", 9. Auflage, S. 185 ff. (1994)).

Die zu oligosacchariden (Glycanen) miteinander verknüpften Monosaccharide sind mit dem Polypeptid entweder über die Aminosäure Asparagin in der typischen Tripeptid-Sequenz-Asparagin-beliebige Aminosäure (ausgenommen Prolin)-Serin oder Threonin oder Cystein-N-glycosidisch verknüpft (N-Glycane) oder sie sind über Serin 0-glycosidisch verknüpft (O-Glycane). In diesen Glycanen können N-Acetyl-D-glycosamin durch N-cyclopropanoyl-D-glucosamin und/oder N-Acetyl-D-galactosamin durch N-cyclopropanoyl-D-galactosamin ersetzt werden.

Im Falle des terminalen Zuckers N-Acetylneuraminsäure findet ein Ersatz durch die N-Cyclopropanoylneuraminsäure statt.

Das von den Zellen synthetisierte und sezernierte EPO enthält durch die Einführung eines oder mehrerer dieser Zukkeranaloga, die einzeln oder als Gemisch dem zellkulturmedium zugesetzt werden können, eine neue Struktur, in der die nativen Monosaccharide durch neue, chemisch modifizierte Monosaccharide ersetzt sind. Die dabei erhaltenen Gesamt-Verbindungen stellen neue Verbindungen dar.

Diese metabolisch modifizierten neuen Erythropoietine weisen neue biologische Eigenschaften auf, insbesondere besitzen sie eine höhere biologische Stabilität. Erklärt wird dies derzeit dadurch, daß es bekannt ist, daß Glycoproteine terminale N-Acetyl-neuraminsäure-Reste enthalten, die mit D-Galactose verknüpft sind (vgl. A. Rosenberg, "Biology of Sialic Acids", Plenum Press, New York und London, Seiten 7-67 (1995)). Ihre Funktion in dieser Verknüpfung besteht darin, diese Galactose vor Zellen und deren Galactose-spezifische Rezeptoren gegen Erkanntwerden zu schützen, was durch die Untersuchungen von G. Ashwell in der obengenannten Schauer-Literaturstelle gezeigt werden konnte. Wenn diese terminalen Galactosen in Glycoproteinen erkannt worden sind, werden die erkannten Glycoproteine, die somit keine N-Acetyl-neuraminsäure mehr tragen, dem intrazellulären Abbau zugeführt. Diese für die biologische Stabilität essentielle N-Acetyl-neuraminsäure unterliegt einem natürlichen Turnover. Er wird im wesentlichen durch spezifische Nenraminidasen (oder Sialidasen) bestimmt, welche die N-Acetyl-neuraminsäure abspalten. Dadurch kommt es zur Alterung dieser Glycoproteine und sie werden dem natürlichen Abbau zugeführt. Diese Neuraminidasen benötigen für ihre volle Aktivität die N-Acetylgruppe in der N-Acetyl-neuraminsäure.

In den erfindungsgemäßen neuen EPO-Verbindungen ist die natürliche N-Acetylgruppe durch homologen cyclishe N-(C₃-C₇)-Acylgruppen ersetzt, beispielsweise durch die N-Propanoylgruppe. Aber auch die obengenannten weiteren N-Acyl- und N-Hydrocarbyl-Gruppen sind wirksame Ersatzgruppen. Der Ersatz der N-Acetyl-neuraminsäure durch die obengenannten homologenen N-Acyl- bzw. N-Hydrocarbyl-Verbindungen führt daher zu einer verminderten Affinität der Neuramminidasen gegenüber diesen modifizierten N-Acyl- bzw. N-Hydrocarbyl-neuraminsäuren. Dadurch wird die modifizierte Neuraminsäure in einer weit geringeren Rate abgespalten, sie bleibt länger mit dem Hydroprotein verbunden und kann länger die erwünschte Schutzfunktion ausüben. Dadurch wird das Glycoprotein biologisch stabiler. Das bedeutet, daß parenteral verabreichtes, in der vorstehend beschriebenen Weise modifiziertes EPO länger im Organismus verbleiben kann. Es ist somit länger aktiv. Dadurch kann die EPO-Dosierung herabgesetzt werden, da das erfindungsgemäß modifizierte EPO die gleiche biologische Aktivität bezüglich der Blutneubildungs-stimulation aufweist wie natives EPO. Außerdem ist das erfindungsgemäß modifizierte EPO stabiler.

Die vorstehende Beschreibung des ablaufenden biologischen Mechanismus bei der Herstellung und Verabreichung von rh Erythropoietin gilt natürlich auch für die anderen erfindungsgemäßen rekombinanten Glycoproteine, d.h. auch für die obengenannten Gerinnungsfaktoren, Interferone, Interleukine, Wachstumsfaktoren, Thrombolytika und Thromboprophylaktika.

Gemäß einem weiteren Aspekt betrifft die vorliegende Erfindung insbesondere die folgenden Verbindungen:
rekombinantes Human-Erythropoietin (rh Erythropoietin) für die Behandlung von Anämien, in dem die natürliche N-Acetyl-Gruppe der Glycan-Reste, insbesondere der terminalen N-Acetylneuraminsäure-Reste, durch einen cyclischen N-(C₃-C₇)-Acylrest, insbesondere einen N-Cyclopropanoylrest einschließlich der ein- oder mehrfach hydroxylierten und/oder ketylierten, Analoga davon ersetzt ist.

Die erfindungsgemäßen neuen rh Erythropoietine weisen vorzugsweise einen Glycan-Rest auf, der ausgewählt wird aus der folgenden Gruppe: 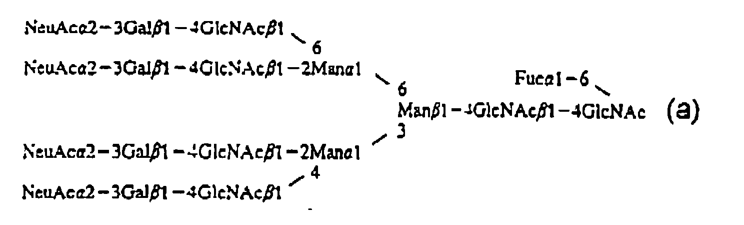 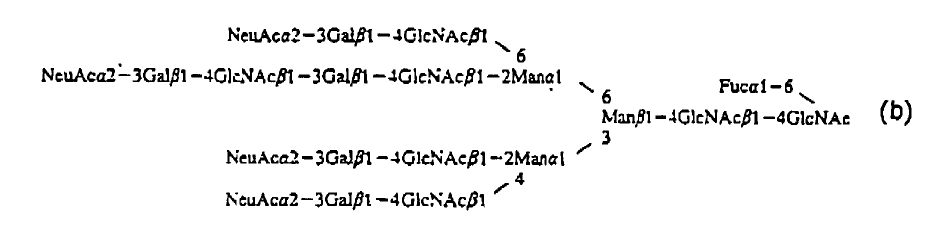 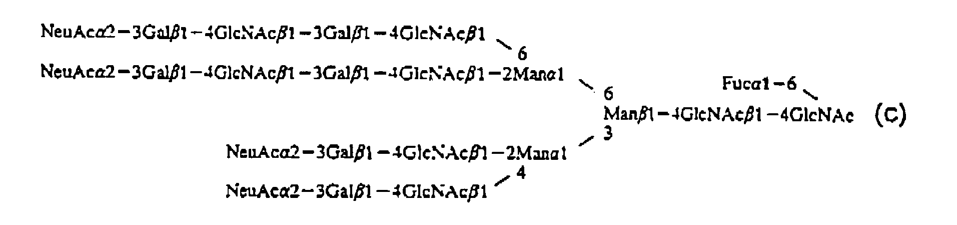 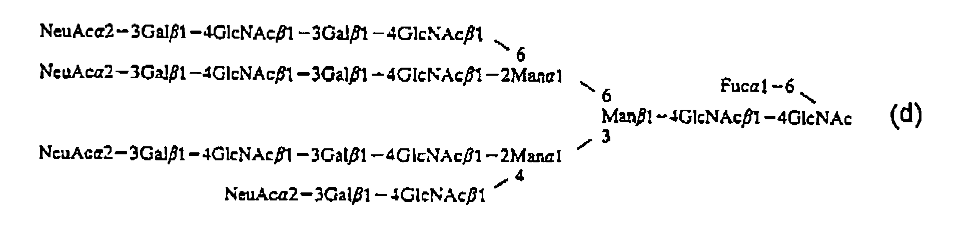 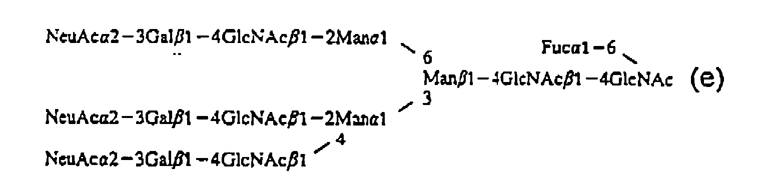 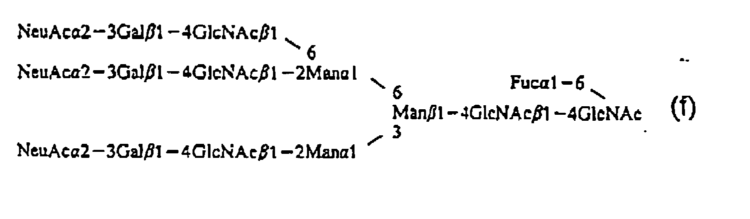 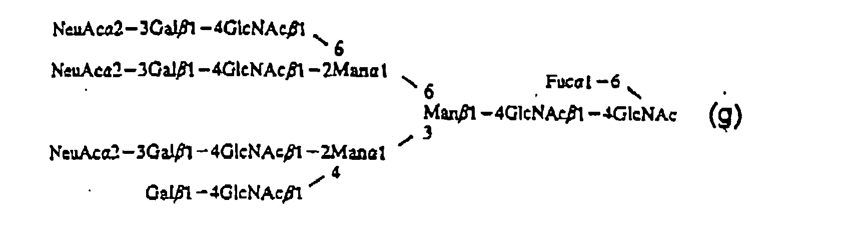 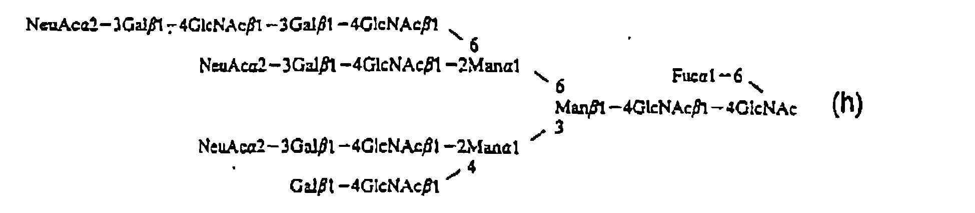 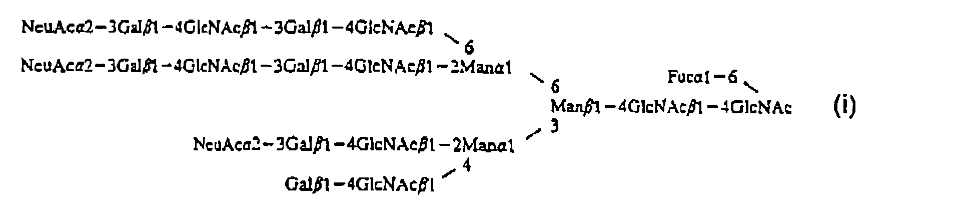 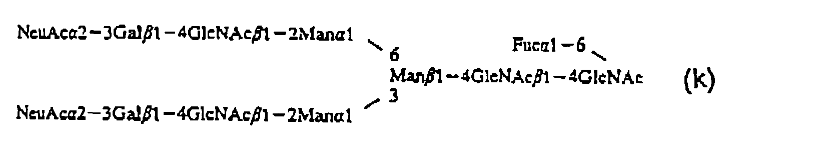 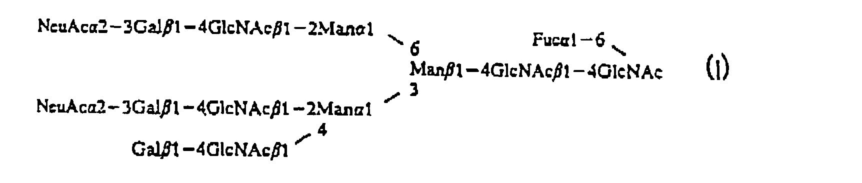 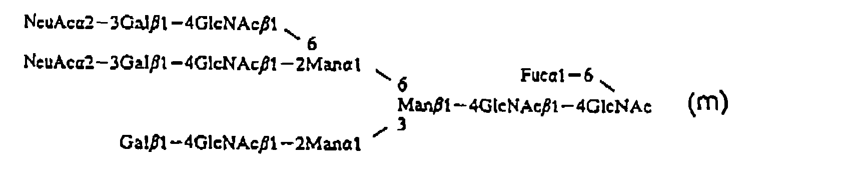 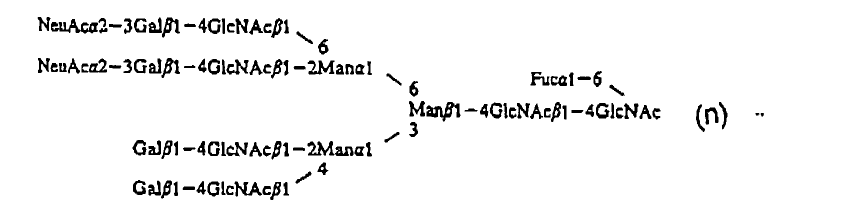 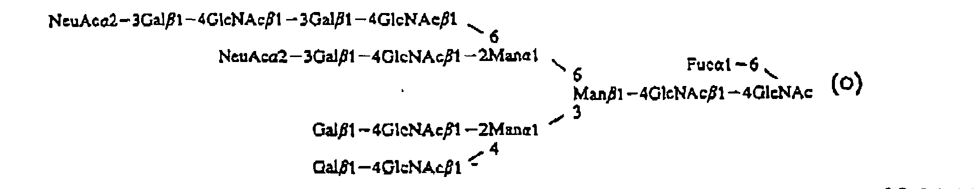 worin Ac in den NeuAc-Resten und/oder ein oder mehrere oder alle Ac in den übrigen Resten jeweils ersetzt ist (sind) durch einen cyclischen (C₃-C₇)-Acylrest, insbesondere durch einen N-Cyclopropanoylrest, einschließlich der ein- oder mehrfach hydroxylierten und/oder ketylierten Analoga davon, ersetzt ist;

Weitere bevorzugte erfindungsgemäße rh Glycoproteine sind folgende:
rh Wachstumsfaktoren, insbesondere rh GCSF und/oder rh GMCSF, für die Behandlung von Erkrankungen des Blutzellen-bildenden Systems, insbesondere der Agranulocytose oder Thrombopenie, vor allem zur Stimulierung des Wachstums von weißen Blutzellen, insbesondere Lymphocyten, Granulocyten und Monocyten, in denen die natürliche N-Acetyl-Gruppe der Glycan-Reste, insbesondere der terminalen N-Acetylneuraminsäure-Reste, durch einen cyclischen N-(C₃-C₇)-Acylrest, insbesondere durch einen N-Cyclopropanoylrest, einschließlich der ein- oder mehrfach hydroxylierten und/oder ketylierten Analoga davon, ersetzt ist;
rh Thrombolytika, insbesondere rh tPA oder rh Urokinase, für die Behandlung von Thromben oder zur Prophylaxe der Thrombenbildung, in denen die natürliche N-Acetyl-Gruppe der Glycan-Reste, insbesondere der terminalen N-Acetylneuraminsäure-Reste, durch einen cyclischen N-(C₃-C₇)-Acylrest, insbesondere durch einen N-Cyclopropanoylrest, einschließlich der ein- oder mehrfach hydroxylierten und/oder ketylierten Analoga davon, ersetzt ist;
rh Thromboprophylaktika, insbesondere rh Antithrombin III, zur Prophylaxe der Thrombenbildung, in denen die natürliche N-Acetyl-Gruppe der Glycan-Reste, insbesondere der terminalen N-Acetylneuraminsäure-Reste, durch einen cyclischen N-(C₃-C₇)-Acylrest, insbesondere durch einen N-Cyclopropanoylrest, einschließlich der ein- oder mehrfach hydroxylierten und/oder ketylierten Analoga davon, ersetzt ist;
rh Gerinnungsfaktoren, insbesondere den rh Faktor VIII und/oder den rh Faktor IX, für die Behandlung von Störungen des Gerinnungssystems, die durch einen erblichen oder erworbenen Mangel an Faktor VIII und/oder Faktor IX bedingt sind, in denen die natürliche N-Acetyl-Gruppe der Glycan-Reste, insbesondere der terminalen N-Acetylneuraminsäure-Reste, durch einen cyclischen N-(C₃-C₇)-Acylrest, insbesondere durch einen N-Cyclopropanoylrest, einschließlich der ein- oder mehrfach hydroxylierten und/oder ketylierten Analoga davon, ersetzt ist;
rh Interferone, insbesondere rh α-Interferon-Verbindungen, für die Behandlung von bösartigen Tumoren, insbesondere Melanomen, und von Autoaggressionskrankheiten, in denen die natürliche. N-Acetyl-Gruppe der Glycan-Reste, insbesondere der terminalen N-Acetylneuraminsäure-Reste, durch cyclischen N-(C₃-C₇)-Acylrest, insbesondere durch einen N-Cyclopropanoylrest, einschließlich der ein- oder mehrfach hydroxylierten und/oder ketylierten Analoga davon, ersetzt ist;
rh Interferone, insbesondere rh β-Interferon-Verbindungen, für die Behandlung aller Formen der multiplen Sklerose und Viruserkrankungen, vor allem der Virushepatitis B, der Virusheptatitis C, der Virusheptatitis D und Virusenzephalitiden, in denen die natürliche N-Acetyl-Gruppe der Glycan-Reste, insbesondere der terminalen N-Acetylneuraminsäure-Reste, durch einen cyclischen N-(C₃-C₇)-Acylrest, insbesondere durch einen N-Cyclopropanoylrest, einschließlich der ein- oder mehrfach hydroxylierten und/oder ketylierten Analoga davon, ersetzt ist;
rh Interferone, insbesondere rh γ-Interferon-Verbindungen, für die Behandlung von bösartigen Tumoren, der Virushepatitis B, der Virusheptatitis C, der Virusheptatitis D und von Virusenzephalitiden, in denen die natürliche N-Acetyl-Gruppe der Glycan-Reste, insbesondere der terminalen N-Acetylneuraminsäure-Reste, durch einen cyclischen N-(C₃-C₇)-Acylrest, insbesondere durch einen N-Cyclopropanoylrest, einschließlich der ein- oder mehrfach hydroxylierten und/oder ketylierten Analoga davon, ersetzt ist; und
rh Interleukine, insbesondere rh IL-2, rh IL-15, rh IL-16 und rh IL-17, für die Behandlung von bösartigen metastasierenden Tumoren, insbesondere Melanomen, in denen die natürliche N-Acetyl-Gruppe der Glycan-Reste, insbesondere der terminalen N-Acetylneuraminsäure-Reste, durch einen cyclischen N-(C₃-C₇)-Acylrest, insbesondere durch einen N-Cyclopropanoylrest, einschließlich der ein- oder mehrfach hydroxylierten und/oder ketylierten Analoga davon, ersetzt ist.

Bei den erfindungsgemäßen neuen Verbindungen handelt es sich insbesondere um solche, in denen die natürliche N-Acetylgruppe des N-Acetyl-D-glucosamins durch N-Cyclopropanoyl ganz oder teilweise ersetzt worden ist durch biochemische Modulation (Biochemical-Engineering) durch Zugabe des jeweils entsprechenden N-Acyl-D-glucosamin- oder N-Acyl-D-galactosamin- oder N-Acyl-neuraminsäure-, insbesondere N-Acyl-D-mannosamin-Derivats, zu dem Kulturmedium in einer Endkonzentration im Medium von 0,001 bis 50,0 mM, insbesondere 0,5 bis 20 mM, speziell 0,5 bis 5 mM, wobei in dem genannten Kulturmedium die D-Glucose ganz oder teilweise durch D-Galactose ersetzt sein kann; und
speziell um solche, in denen die natürliche D-Galactose durch 2-Desoxy-D-galactose ganz oder teilweise ersetzt worden ist durch biochemische Modulation (Biochemical-Engineering) durch Zugabe von 2-Desoxy-D-galactose zu dem Kulturmedium in einer Endkonzentration im Medium von 0,001 bis 50 mM, insbesondere 0,5 bis 20 mM, speziell 0,5 bis 5 mM.

Gemäß einem weiteren Aspekt betrifft die vorliegende Erfindung pharmazeutische Mittel, die als Wirkstoff mindestens ein rh Glucosamin, wie es vorstehend definiert worden ist, gegebenenfalls in Kombination mit anderen Wirkstoffen sowie üblichen pharmazeutischen Trägern und/oder Hilfsstoffen, enthalten.

Vorzugsweise enthalten die erfindungsgemäßen pharmazeutischen Mittel als Wirkstoff ein oder mehrere rekombinante Glycoproteine, insbesondere rekombinante Human-Glyco teine oder ein Gemisch unterschieldich modifizierter cyclischer N-(C₃-C₇)-Acyl- Derivate davon.

Die erfindungsgemäßen pharmazeutischen Mittel enthalten den rh Glycoprotein-Wirkstoff, der vorzugsweise am Monosaccharid ein- oder mehrfach acyliert, insbesondere acetyliert, ist, vorzugsweise in einer Menge von 0,001 bis 50 Gew.-%, vorzugsweise von 0,1 bis 20 Gew.-%, speziell von 2 bis 10 Gew.-%.

Besonders bevorzugte pharmazeutische Mittel der Erfindung sind solche, die als rh Glyeoprotein-Wirkstoff enthalten:
rekombinantes Human-Erythropoietin oder Derivate davon, wie sie vorstehend definiert sind;
rh Wachstumsfaktoren, insbesondere rh CGSF und/oder rh GMCSF, wie sie vorstehend definiert sind;
rh Thrombolytika, insbesondere rh tPA oder rh Urokinase, wie sie vorstehend definiert sind;
rh Thromboprophylaktika, insbesondere rh Antithrombin III, wie sie vorstehend definiert sind;
rh Gerinnungsfaktoren, insbesondere den rh Faktor VIII und/oder den rh Faktor IX, wie sie vorstehend definiert sind;
rh Interferone, insbesondere rh α-, β- und γ-interferone, wie sie vorstehend definiert worden sind; oder
rh Interleukine, insbesondere rh IL-2, rh IL-15, rh IL-16 und rh IL-17, wie sie vorstehend definiert worden sind.

Gemäß einem weiteren Aspekt betrifft die vorliegende Erfindung
die Verwendung von rekombinantem Human-Erythropoietin und seinen Derivaten, wie es vorstehend definiert worden ist, zur Herstellung eines Arzneimittels für die Behandlung von Anämien, insbesondere renal bedingten Anämien (z.B. in einer Dosierung von 0,001 bis 200 mg/kg Körpergewicht);
die Verwendung der rh Wachstumsfaktoren, wie sie vorstehend definiert worden sind, zur Herstellung eines Arzneimittels für die Behandlung von Erkrankungen des Blutzellen-bildenden Systems, insbesondere der Agranulozytose oder Thrombopenie, vor allem zur Stimulierung des Wachstums von weißen Blutzellen, insbesondere Lymphozyten, Granulozyten und Monozyten, (z.B. in einer Dosierung von 0,001 bis 1,0 mg/kg Körpergewicht);
die Verwendung der rh Thrombolytika, wie sie vorstehend definiert worden sind, zur Herstellung eines Arzneimittels für die Behandlung von Thromben oder zur Prophylaxe der Thrombenbildung (z.B. in einer Dosierung von 1 bis 200 mg/kg Körpergewicht, vorzugsweise von 50 bis 100 mg/kg Körpergewicht);
die Verwendung der rh Thromboprophylaktika, wie sie vorstehend definiert worden sind, zur Herstellung eines Arzneimittels zur Prophylaxe der Thrombenbildung (z.B. in einer Dosierung von 1 bis 200 mg/kg Körpergewicht, vorzugsweise von 50 bis 100 mg/kg, Körpergewicht);
die Verwendung der rh Gerinnungsfaktoren, wie sie vorstehend definiert worden sind, zur Herstellung eines Arzneimittels für die Behandlung von Störungen des Gerinnungssystems, die durch einen erblichen oder erworbenen Mangel an Faktor VIII und/oder Faktor IX bedingt sind, in einer Dosierung von 0,001 bis 200, vorzugsweise von 0,1 bis 50 mg/kg Körpergewicht;
die Verwendung der rh α-Interferone, wie sie vorstehend definiert worden sind zur Herstellung eines Arzneimittels für die Behandlung von bösartigen Tumoren, insbesondere Melanomen, und von Autoaggressions-Krankheiten (z.B. in einer Dosierung von 0,001 bis 10 µg/kg Körpergewicht, insbesondere von 0,01 bis 0,1 µg/kg Körpergewicht);
die Verwendung der rh β-Interferone, wie sie vorstehend definiert worden sind, zur Herstellung eines Arzneimittels für die Behandlung aller Formen der multiplen Sklerose und von Viruserkrankungen, vor allem der Virushepatitis B, der Virusheptatitis c, der Virusheptatitis D und Virusenzephalitiden (z.B. in einer Dosierung von 0,001 bis 10 µg/kg Körpergewicht, insbesondere von 0,01 bis 0,1 µg/kg Körpergewicht);
die Verwendung der rh γ-Interferone wie sie vorstehend definiert worden sind, zur Herstellung eines Arzneimittels für die Behandlung von bösartigen Tumoren, der Virushepatitis B, der Virusheptatitis c, der Virusheptatitis D und von Virusenzephalitiden (z.B. in einer Dosierung von 0,001 bis 0,1 µg/kg Körpergewicht, insbesondere von 0,01 bis 0,1 µg/kg Körpergewicht); und
die Verwendung der rh Interleukine, wie sie vorstehend definiert worden sind, zur Herstellung eines Arzneimittels für die Behandlung von bösartigen metastasierenden Tumoren, insbesondere Melanomen, (z.B. in einer Dosierung von 0,001 bis 200 µg/kg Körpergewicht).

Die erfindungsgemäßen rh Gluooproteine werden vorzugsweise in einer Form verabreicht, in der die OH- bzw. OR-Gruppen ihres Monosaccharid-Restes ein- oder mehrfach acyliert, vorzugsweise acetyliert, ist, wobei in diesem Fall für die entsprechend acylierten bzw. acetylierten Derivate die Dosierung auf die Hälfte der bei den obengenannten bevorzugten Verwendungen angegebenen Dosierungen herabgesetzt werden kann.

## Patentansprüche

1. Rekombinante Glycoproteine der allgemeinen Formel (I) 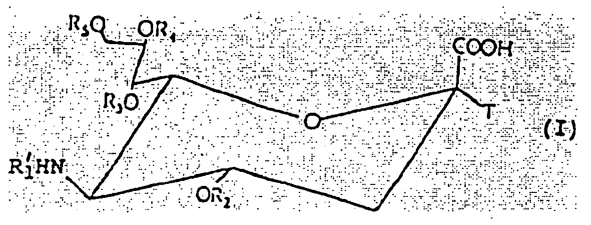 worin bedeuten:
R'₁ einen cyclischen (C₃-C₇)-Acylrest, der eine oder mehrere Hydroxyl- und/oder Carbonylgruppen aufweisen kann,
R₂, R₃, R₄ und R₅, die gleich oder verschieden sein können, jeweils Wasserstoff, einen linearen oder verzweigten C₁₋₂₀-Alkylrest; einen linearen oder verzweigten C₃. ₂₀-Alkenylrest; einen linearen oder verzweigten C₃₋₂₀-Alkinylrest; einen C₄₋₂₀-Alkenyl- oder Alkinylrest mit 2 oder mehr Doppelbindungen bzw. Dreifachbindungen; einen C₆₋₂₀-Arylrest; einen linearen oder verzweigten, gesättigten oder ein- oder mehrfach ungesättigten Acylrest (-CO-R₁) mit insgesamt 1 bis 20 Kohlenstoffatomen, der eine oder mehrere Hydroxylgruppen aufweisen kann; einen C₆₋₂₀-Aroylrest; einen Carbonylamidrest der Formel -CONH₂ oder - CONHR₁; einen linearen oder verzweigten, gesättigten oder ein- oder mehrfach ungesättigten Thioacylrest (-CS-R₁) mit insgesamt 1 bis 20 Kohlenstoffatomen; oder einen Thiocarbamidrest der Formel -CS-NH₂ oder -CS-NHR₁; wobei R₁ jeweils für Wasserstoff oder einen linearen oder verzweigten, gesättigten oder ein- oder mehrfach ungesättigten C₁₋₂₀-Alkylrest steht und wobei jeder der vorgenannten Reste außer H eine oder mehrere Halogen-, Hydroxy-, Epoxy-, Amino-, Mercaptan-, Phenyl-, Phenol- oder Benzylgruppen als Substituent(en) aufweisen kann und
T einen Mono-, Di- oder Oligosaccharidrest mit bis zu 40 glykosidisch miteinander verknüpften Zuckerresten, die Furanose- und/oder Pyranoseringe darstellen, und 5 bis 230 Kohlenstoffatome enthalten und N- oder 0-glycosidisch an Polypeptide gebunden sind.

2. Glycoproteine nach Anspruch 1, wobei in der allgemeinen Formel (I):
R₂, R₃, R₄ und R₅, die gleich oder verschieden sein können, jeweils Wasserstoff, einen linearen oder verzweigten Alkylrest mit 1 bis 7 Kohlenstoffatomen; einen linearen oder verzweigten Alkenylrest mit 3 bis 10 Kohlenstoffatomen; einen linearen oder verzweigten Alkinylrest mit 3 bis 10 Kohlenstoffatomen; einen Alkenyl- bzw. Alkinylrest mit 2 oder mehr Doppelbindungen bzw. Dreifachbindungen mit 7 bis 12 Kohlenstoffatomen; einen Phenylrest; einen linearen oder verzweigten, gesättigten oder ein- oder mehrfach ungesättigten Acylrest (-CO-R₁) mit insgesamt 1 bis 7 Kohlenstoffatomen, insbesondere Acetylrest, der eine oder mehrere Hydroxylgruppen aufweisen kann; einen Aroylrest mit 6 bis 10 Kohlenstoffatomen; einen Carbonylamidrest der Formel -CONH₂ oder -CONHR₁; einen linearen oder verzweigten, gesättigten oder ein- oder mehrfach ungesättigten Thioacylrest (-CS-R₁) mit insgesamt 1 bis 7 Kohlenstoffatomen; oder einen Thiocarbamidrest der Formel -CS-NH₂ oder - CS-NHR₁; wobei R₁ jeweils für Wasserstoff oder einen linearen oder verzweigten, gesättigten oder ein- oder mehrfach ungesättigten Alkylrest mit 1 bis 20 Kohlenstoffatomen steht und jeder der vorgenannten Reste außer H gegebenenfalls ein- oder mehrfach substituiert sein kann durch Fluor, Chlor, Brom oder Iod, Hydroxy-, Epoxy-, Amino-, Mercaptan-, Phenyl-, Phenol- oder Benzylgruppen.

3. Verbindungen nach Anspruch 1 oder 2, wobei in der allgemeinen Formel (I) T für einen Saccharidrest mit N-Glycan-Struktur der Formel (Ia) steht: 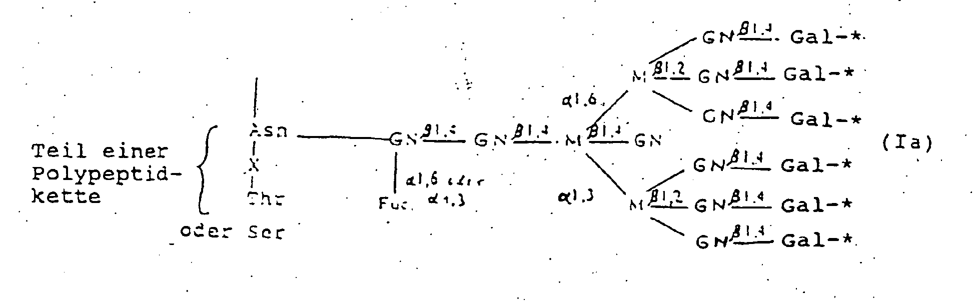 worin bedeuten:
Gal = Galactose,
GN = N-Acetyl-D-glucosamin,
M = D-Mannose,
Fuc = Fucose,
Asn = Asparagin,
X = eine beliebige Aminosaure aufier Prolin,
Thr = Threonin,
Ser = Serin,
* = T-Verknüpfungsstelle (1 bis 6 Molekülreste),
wobei beide peripheren Reste M durch 1 bis 3 Trisaccharide substituiert sein können; oder für einen Saccharidrest mit O-Glycan-Struktur der allgemeinen Formel (Ib) steht: 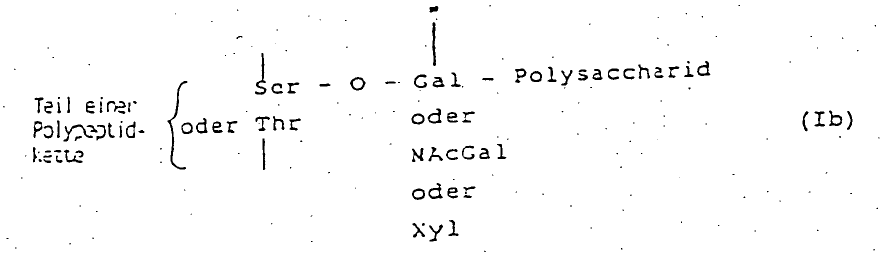 worin bedeuten:
Gal = Galactose
Thr = Threonin
30 Ser = Serin
Xyl = Xylose
NAcGal = N-Acetyl-galactosamin
* = T-Verknüpfungsstelle,
wobei in den obigen Formeln (Ia) und (Ib) die Galactose (Gal) ersetzt sein kann durch 2-Desoxygalactose oder 2-Desoxy-2-halogenid(F, Cl, Br, I)-galactose.

4. Verbindungen nach mindestens einem der Ansprüche 1 bis 3, wobei dann, wenn T einen Saccharidrest mit N-Glycanstruktur oder einen Saccharidrest mit O-Glycanstruktur darstellt, GN für einen Rest der allgemeinen Formel (II) steht: 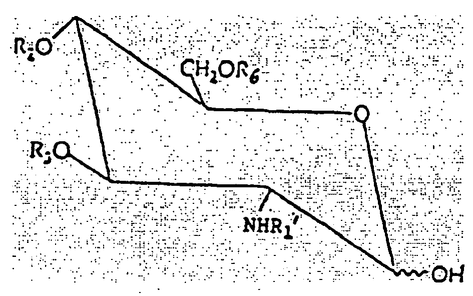 in der R'₁, R₂ und R₃ die vorstehend angegebenen Bedeutungen haben, R₆ die gleichen Bedeutungen wie R₂ und R₃ hat und -NHR'₁ neben der äquatorialen Position auch die axiale Position einnehmen kann, und wobei außerdem bei äquatorialer Position von -NHR'₁ -OR₂ in axialer Position stehen kann, und in der eine oder mehrere oder alle der Gruppen -OR₂, -OR₃, -OR₆ und/oder die freie OH-Gruppe acyliert sein können durch einen linearen oder verzweigten, gesättigten oder ein- oder mehrfach ungesättigten Acylrest (-CO-R₁) mit insgesamt 1 bis 20, vorzugsweise 1 bis 7 Kohlenstoffatomen, vorzugsweise durch den Acetylrest, der eine oder mehrere Hydroxylgruppen aufweisen kann.

5. Verbindungen nach mindestens einem der Anspruche 1 bis 4, wobei R₁ bis R₆ für H oder CH₃ oder (C₁-C₇)-Acyl, insbesondere Acetyl, stehen.

6. Verbindungen nach mindestens einem der Ansprüche 1 bis 5, wobei der (C₃-C₇)-Acylrest von R'₁ ein Cyclopropanoylrest ist, der eine oder mehrere Hydroxyl- und/oder Carbonylgruppen aufweisen kann.

7. Verbindung nach mindestens einem der Ansprüche 1 bis 6, wobei es sich bei dem rh Glycoprotein um rh Differenzierungsfaktoren, insbesondere rh Erythropoietin; um rh Wachstumsfaktoren, insbesondere rh CSF (Colony-Stimulating-Factor), rh GMCSF (Granulocyten-Monocyten-Stimulationsfaktor); um rh Thrombolytika, insbesondere rh Tissue-Plasminogen-Activator (tPA) und rh Urokinase; um rh Thromboprophylaktika, insbesondere rh Antithrombin III; um rh Gerinnungsfaktoren, insbesondere den rh Faktor VIII und den rh Faktor IX; um rh Interferone, insbesondere rh α-, β- und γ-Interferone; und um rh Interleukine, insbesondere rh IL-2, rh IL-15, rh IL-16 und rh IL-17, handelt.

8. Verbindungen nach Anspruch 7, wobei es sich dabei um rekombinantes Human-Erythropoietin (rh Erythropoietin) für die Behandlung von Anämien handelt, in dem die natürliche N-Acetyl-Gruppe der Glycanreste, insbesondere der terminalen N-Acetylneuraminsäure-Reste, durch einen wie in Anspruch 6 definierten N-(C₃-C₇)-Acylrest ersetzt ist.

9. Verbindungen nach Anspruch 8, die einen der folgenden Glycanreste aufweisen: 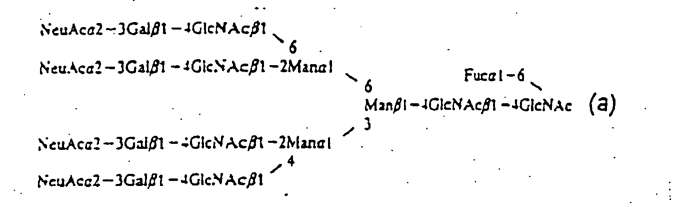 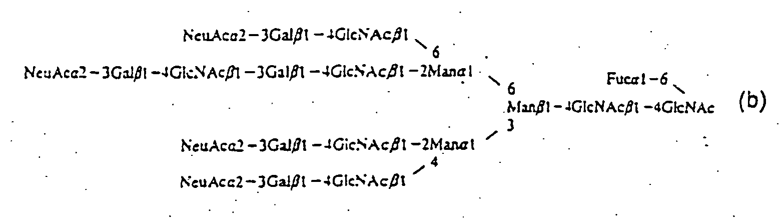 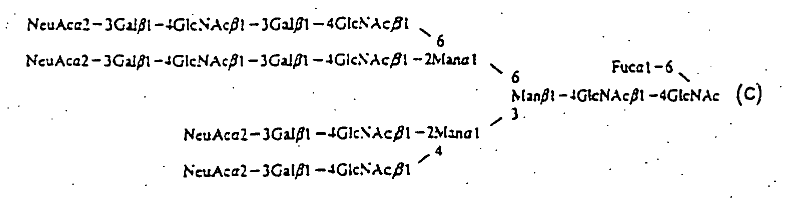 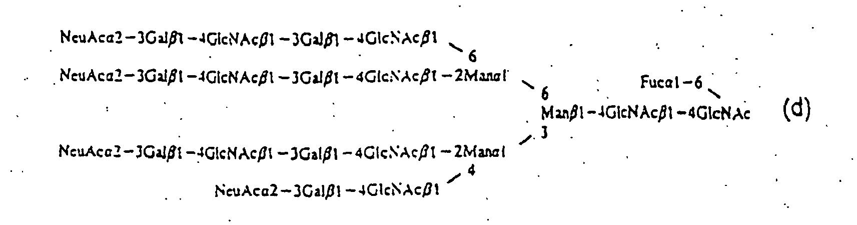 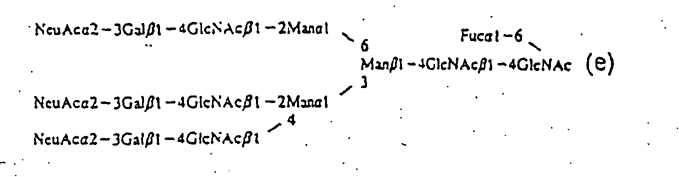 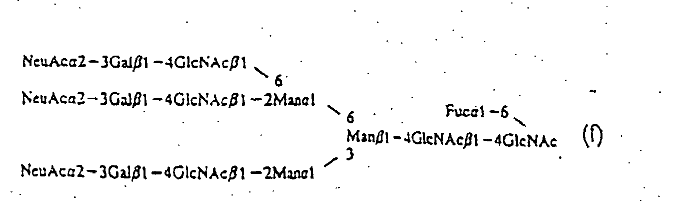 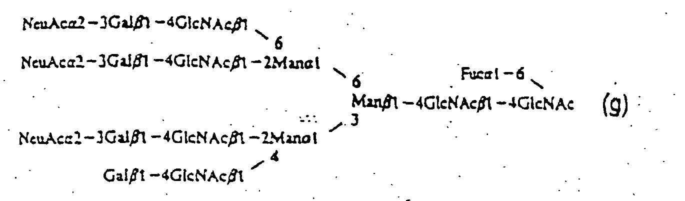 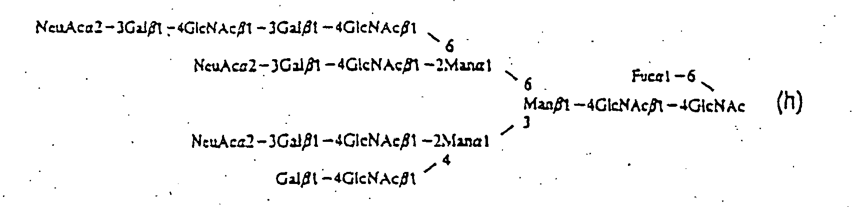 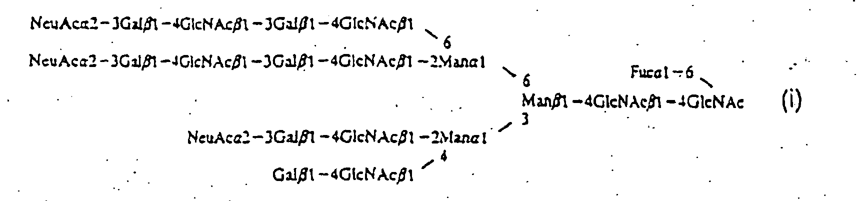 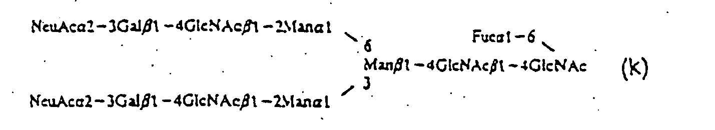 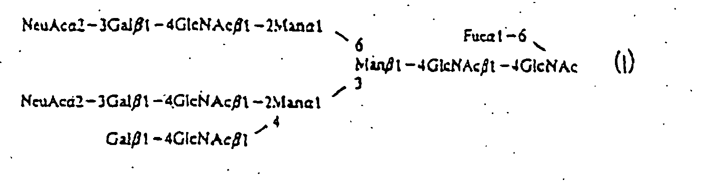 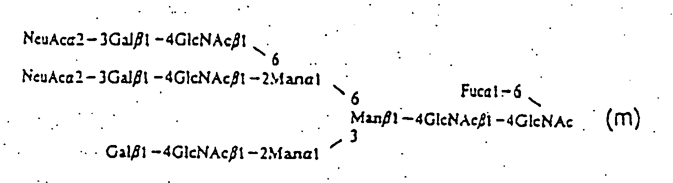 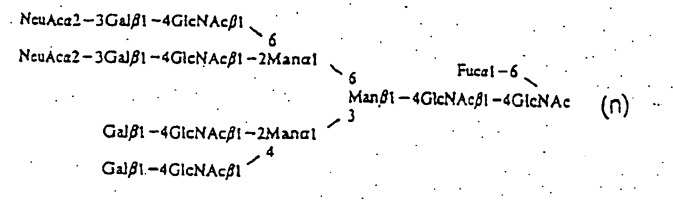 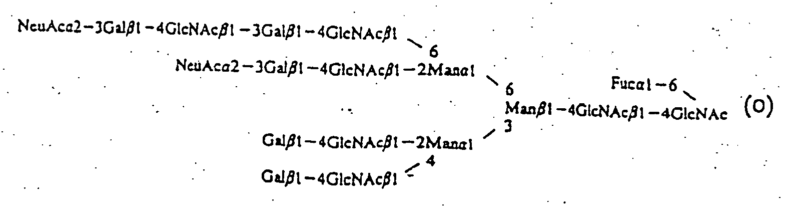 worin Ac in den NeuAc-Resten und/oder ein oder mehrere oder alle Ac in den übrigen Resten jeweils ersetzt ist (sind) durch einen wie in Anspruch 6 definierten (C₃-C₇)-Acylrest.

10. Verbindungen nach Anspruch 7, wobei es sich um rh Wachstumsfaktoren, insbesondere rh GCSF und/oder rh GMCSF, für die Behandlung von Erkrankungen des Blutzellen-bildenden Systems, insbesondere der Agranulocytose oder Thrombopenie, vor allem zur Stimulierung des Wachstums von weißen Blutzellen, insbesondere Lymphocyten, Granulocyten und Monocyten, handelt, in denen die natürliche N-Acetyl-Gruppe der Glycan-Reste, insbesondere der terminalen N-Acetylneuraminsaure-Reste, durch einen wie in Anspruch 6 definierten N-(C₃-C₇)-Acylrest ersetzt ist.

11. Verbindungen nach Anspruch 7, wobei es sich um rh Thrombolytika, insbesondere rh tPA oder rh Urokinase, für die Behandlung von Thromben oder zur Prophylaxe der Thrombenbildung handelt, in denen die natürliche N-Acetyl-Gruppe der Glycan-Reste, insbesondere der terminalen N-Acetylneuraminsaure-Reste, durch einen wie in Anspruch 6 definierten N- (C₃-C₇) -Acylrest ersetzt ist.

12. Verbindungen nach Anspruch 7, wobei es sich um rh Thromboprophylaktika, insbesondere rh Antithrombin III, zur Prophylaxe der Thrombenbildung handelt, in denen die natürliche N-Acetyl-Gruppe der Glycan-Reste, insbesondere der terminalen N-Acetylneuraminsäure-Reste, durch einen wie in Anspruch 6 definierten N-(C₃-C₇)-Acylrest ersetzt ist.

13. Verbindungen nach Anspruch 7, wobei es sich um rh Gerinnungsfaktoren, insbesondere den rh Faktor VIII und/oder den rh Faktor IX, für die Behandlung von. Störungen des Gerinnungssystems, die durch einen erblichen oder erworbenen Mangel an Faktor VIII und/oder Faktor IX bedingt sind, handelt, in denen die natürliche N-Acetyl-Gruppe der Glycan-Reste, insbesondere der terminalen N-Acetylneuraminsäure-Reste, durch einen wie in Anspruch 6 definierten N-(C₃-C₇)-Acylrest ersetzt ist.

14. Verbindungen nach Anspruch 7, wobei es sich um rh Interferone, insbesondere rh α-Interferon-Verbindungen, für die Behandlung von bösartigen Tumoren, insbesondere Melanomen, und von Autoaggressionskrankheiten handelt, in denen die natürliche N-Acetyl-Gruppe der Glycan-Reste, insbesondere der terminalen N-Acetylneuraminsäure-Reste, durch einen wie in Anspruch 6 definierten N-(C₃-C₇)-Acylrest ersetzt ist.

15. Verbindungen nach Anspruch 7, wobei es sich um rh Interferone, insbesondere rh β-Interferon-Verbindungen, für die Behandlung aller Formen der multiplen Sklerose und Viruserkrankungen, vor allem der Virushepatitis B, der Virushepatitis C, der Virushepatitis D und Virusenzephalitiden, handelt, in denen die natürliche N-Acetyl-Gruppe der Glycan-Reste, insbesondere der terminalen N-Acetylneuraminsäure-Reste, durch einen wie in Anspruch 6 definierten N-(C₃-C₇)-Acylrest ersetzt ist.

16. Verbindungen nach Anspruch 7, wobei es sich um rh Interferone, insbesondere rh γ-Interferon-Verbindungen, für die Behandlung von bösartigen Tumoren, der Virushepatitis B, der Virushepatitis C, der Virushepatitis D und von Virusenzephalitiden, handelt, in denen die natürliche N-Acetyl-Gruppe der Glycan-Reste, insbesondere der terminalen N-Acetylneuraminsäure-Reste, durch einen wie in Anspruch 6 definierten N-(C₃-C₇)-Acylrest ersetzt ist.

17. Verbindungen nach Anspruch 7, wobei es sich um rh Interleukine, insbesondere rh IL-2, rh IL-15, rh IL-16 und rh IL-17, für die Behandlung von bösartigen metastasierenden Tumoren, insbesondere Melanomen, handelt, in denen die natürliche N-Acetyl-Gruppe der Glycan-Reste, insbesondere der terminalen N-Acetylneuraminsäure-Reste, durch einen wie in Anspruch 6 definierten N-(C₃-C₇)-Acylrest ersetzt ist.

18. Verbindungen nach mindestens einem der Ansprüche 7 bis 17, in denen die natürliche N-Acetylgruppe des N-Acetyl-D-glucosamins durch N-Cyclopropanoyl ganz oder teilweise ersetzt worden ist durch biochemische Modulation (Biochemical-Engineering) durch Zugabe des jeweils entsprechenden N-Acyl-D-glucosamin- oder N-Acyl-D-galactosamin- oder N-Acyl-neuraminsäure-, insbesondere N-Acyl-D-mannosamin-Derivats zu dem Kulturmedium in einer Endkonzentration im Medium von 0,001 bis 50,0 mM, insbesondere 0,5 bis 20 mM, speziell 0,5 bis 5 mM, wobei in dem genannten Kulturmedium die D-Glucose ganz oder teilweise durch D-Galactose ersetzt sein kann.

19. Verbindungen nach mindestens einem der Ansprüche 7 bis 18, **dadurch gekennzeichnet, dass** die natürliche D-Galactose durch 2-Desoxy-D-galactose ganz oder teilweise ersetzt worden ist durch biochemische Modulation (Biochemical-Engineering) durch Zugabe von 2. Desoxy-D-galactose zu dem Kulturmedium in einer Endkonzentration im 10 Medium von 0,001 bis 50 mM, insbesondere 0,5 bis 20 mM, speziell 0,5 bis 5 mM.

20. Verfahren zur Herstellung von rekombinanten Glycoproteinen nach mindestens einem der Ansprüche 1 bis 19 aus einem oder mehreren rekombinaten Glycoproteinen, insbesondere rekombinante Human-Glycoproteine, enthaltendes Material, das aus Zellkulturüberständen gewonnen wurde, wobei eukaryote Zellen vorzugsweise in einem Kulturmedium, das als. Glycanvorläufer ein N-(C₃-C₇)-acyliertes Monosaccharid für die Biosynthese der Aminozucker in einer Konzentration von 0,001 bis 50 mMol/l enthält, bei einer Temperatur von 18 bis 40°C und bei einem pH-Wert von 6,8 bis 8,2 kultiviert und das dabei erhaltene N-Acylderivat des Glycoproteins in an sich bekannter Weise aus dem Kulturrückstand abtrennt und gewinnt und gegebenenfalls auf an sich bekannte Weise an den OH- bzw. OR-Gruppen des Monosaccharids ein- oder mehrfach oder vollständig acyliert.

21. Verfahren nach Anspruch 20, wobei die eukaryoten Zellen tierischen Zellen, vorzugsweise nichttransformierte CHO-Zellen oder COS7-Zellen oder Nalm-Zellen oder Fibroblasten sind.

22. Verfahren nach Anspruch 20 oder 21, wobei als Kulturmedium ein FCS-, RPMI 1640-, HAT-, Eagle-MEM-, Dulbecco-MEM- und/oder Glasgow-MEM-Medium verwendet wird (werden).

23. Verfahren nach Anspruch 20, 21 oder 22 wobei als Glycanvorläufer N-Isopropanoyl, -D-glucosamin, -D-galactosamin oder -neuraminsäure, insbesondere -D-mannosamin, oder eine Mischung davon verwendet wird.

24. Pharmazeutisches Mittel, das als Wirkstoff mindestens eine Verbindung nach einem oder mehreren der Ansprüche 1 bis 19, gegebenenfalls in Kombination mit anderen Wirkstoffen sowie üblichen pharmazeutischen Trägern und/oder Hilfsstoffen, enthält.

25. Pharmazeutisches Mittel nach Anspruch 24, **dadurch gekennzeichnet, dass** es als Wirkstoff eine oder mehrere rekombinante Human-Glycoproteine, oder ein Gemisch unterschiedlich modifizierter N-(C₃-C₇)-Acyl-Derivate enthält.

26. Pharmazeutisches Mittel nach Anspruch 24 oder 25, dass den (die) vorzugsweise am Monosaccharid ein- oder mehrfach acylierten, vorzugsweise acetylierten, Wirkstoff (Wirkstoffe) in einer Menge von 0,001 bis 50 Gew.-%, vorzugsweise von 0,1 bis 20 Gew.-%, insbesondere von 2 bis 10 Gew.-%, enthält.

27. Pharmazeutisches Mittel nach den Ansprüchen 24 bis 26, das als Wirkstoff die Verbindung nach den Ansprüchen 7 bis 9 enthält.

28. Pharmazeutisches Mittel nach den Ansprüchen 24 bis 26, das als Wirkstoff die Verbindung nach Anspruch 10 enthält.

29. Pharmazeutisches Mittel nach den Ansprüchen 24 bis 26, das als Wirkstoff die Verbindung nach Anspruch 11 enthält.

30. Pharmazeutisches Mittel nach den Ansprüchen 24 bis 26, das als Wirkstoff die Verbindung nach Anspruch 12 enthält.

31. Pharmazeutisches Mittel nach den Ansprüchen 24 bis 26, das als Wirkstoff die Verbindung nach Anspruch 13 enthält.

32. Pharmazeutisches Mittel nach den Ansprüchen 24 bis 26, das als Wirkstoff die Verbindung nach Anspruch 14 enthält.

33. Pharmazeutisches Mittel nach den Ansprüchen 24 bis 26, das als Wirkstoff die Verbindung nach Anspruch 15 enthält.

34. Pharmazeutisches Mittel nach den Ansprüchen 24 bis 26, das als Wirkstoff die Verbindung nach Anspruch 16 enthält.

35. Pharmazeutisches Mittel nach den Ansprüchen 24 bis 26, das als Wirkstoff die Verbindungen nach Anspruch 17 enthält.

36. Verwendung der Verbindungen nach Anspruch 8 und 9 zur Herstellung eines Arzneimittels für die Behandlung von Anämien, insbesondere renal bedingten Anämien.

37. Verwendung der Verbindungen nach Anspruch 10 zur Herstellung eines Arzneimittels für die Behandlung von Erkrankungen des Blutzellen-bildenden Systems, insbesondere der Agranulozytose oder Thrombopenie, vor allem zur Stimulierung des Wachstums von weißen Blutzellen, insbesondere Lymphozyten, Granulozyten und Monozyten.

38. Verwendung der Verbindungen nach Anspruch 11 zur Herstellung eines Arzneimittels für die Behandlung von Thromben oder zur Prophylaxe der Thrombenbildung.

39. Verwendung der Verbindungen nach Anspruch 12 zur Herstellung eines Arzneimittels zur Prophylaxe der Thrombenbildung.

40. Verwendung der Verbindungen nach Anspruch 13 zur Herstellung eines Arzneimittels für die Behandlung von Störungen des Gerinnungssystems, die durch einen erblichen oder erworbenen Mangel an Faktor VIII und/oder Faktor IX bedingt sind.

41. Verwendung der Verbindungen nach Anspruch 14 zur Herstellung eines Arzneimittels für die Behandlung von bösartigen Tumoren, insbesondere Melanomen, und von Autoaggressions-Krankheiten.

42. Verwendung der Verbindungen nach Anspruch 15 zur Herstellung eines Arzneimittels für die Behandlung aller Formen der multiplen Sklerose und von Viruserkrankungen, vor allem der Virushepatitis B, der Virushepatitis C, der Virushepatitis D und Virusenzephalitiden.

43. Verwendung der Verbindungen nach Anspruch 16 zur Herstellung eines Arzneimittels für die Behandlung von bösartigen Tumoren, der Virushepatitis B, der Virushepatitis C, der Virushepatitis D und von Virusenzephalitiden.

44. Verwendung der Verbindungen nach Anspruch 17 zur Herstellung eines Arzneimittels für die Behandlung von bösartigen metastasierenden Tumoren, insbesondere Melanomen.

45. Verwendung der Verbindungen nach den Ansprüchen 1 bis 19 in ihrer an den OH- bzw. OR-Gruppen des Monosaccharid-Restes ein- oder mehrfach acylierten, vorzugsweise acetylierten, Form zur Herstellung eines Arzneimittels zu den in den Ansprüchen 36 bis 44 angegebenen Zwecken.

## Claims

1. Recombinant glycoproteins having the general formula (I) 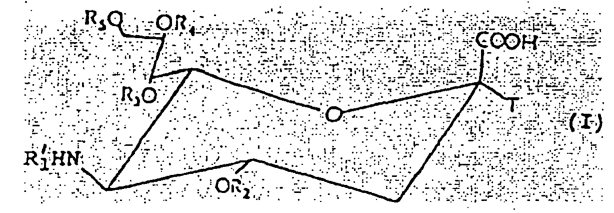 wherein:
R'₁ is a cyclic (C₃-C₇) acyl radical that may have one or more hydroxy and/or carbonyl groups;
R₂, R₃, R₄ and R₅, which can be the same or different, each are hydrogen, a linear or branched C₁₋₂₀ alkyl radical; a linear or branched C₃₋₂₀ alkenyl radical; a linear or branched C₃₋₂₀ alkynyl radical; a C₄₋₂₀ alkenyl or alkynyl radical having 2 or more double bonds or triple bonds, respectively; a C₆-₂₀ aryl radical; a linear or branched, saturated or mono- or polyunsaturated acyl radical (-CO-R₁) having in total 1 to 20 carbon atoms and optionally having one or more hydroxy groups; a C₆₋₂₀ aroyl radical; a carbonylamide radical of formula -CONH₂ or -CONHR₁; a linear or branched, saturated or mono- or polyunsaturated thioacyl radical (-CS-R₁) having in total 1 to 20 carbon atoms; or a thiocarbamide radical of formula -CS-NH₂ or -CS-NHR₁; where R₁ each represents hydrogen, a linear or branched, saturated or mono- or polyunsaturated C₁₋₂₀ alkyl radical, and where each of the above-mentioned radicals with the exception of H optionally can have one or more of halogen, hydroxy, epoxy, amino, mercaptan, phenyl, phenol or benzyl groups as substituents; and
T is a mono- or di- or oligosaccharide radical having up to 40 glycosidically linked sugar radicals that are furanose and/or pyranose rings and contain from 5 to 230 carbon atoms and are N- or O-glycosidically bound to polypeptides.

2. The glycoproteins of claim 1, wherein in general formula (I):
R₂, R₃, R₄ and R₅, which can be the same or different, each represent hydrogen, a linear or branched alkyl radical having 1 to 7 carbon atoms; a linear or branched alkenyl radical having 3 to 10 carbon atoms; a linear or branched alkynyl radical having 3 to 10 carbon atoms; an alkenyl or alkynyl radical having 2 or more double bonds or triple bonds, respectively, and having 7 to 12 carbon atoms; a phenyl radical; a linear or branched, saturated or mono- or polyunsaturated acyl radical (-CO-R₁) having in total 1 to 7 carbon atoms, in particular an acetyl radical, and optionally having one or more hydroxy groups; an aroyl radical having 6 to 10 carbon atoms; a carbonylamide radical of formula -CONH₂ or -CONHR₁; a linear or branched, saturated or mono- or polyunsaturated thioacyl radical (-CS-R₁) having in total 1 to 7 carbon atoms; or a thiocarbamide radical of formula -CS-NH₂ or -CS-NHR₁; where R₁ each represents hydrogen or a linear or branched, saturated or mono- or polyunsaturated alkyl radical having 1 to 20 carbon atoms, and where each of the above-mentioned radicals with the exception of H optionally can be mono- or polysubstituted by fluorine, chlorine, bromine or iodine, hydroxy, epoxy, amino, mercaptan, phenyl, phenol or benzyl groups.

3. The compounds of claim 1 or 2, wherein in general formula (I): T represents a saccharide radical having an N-glycan structure of formula (Ia): 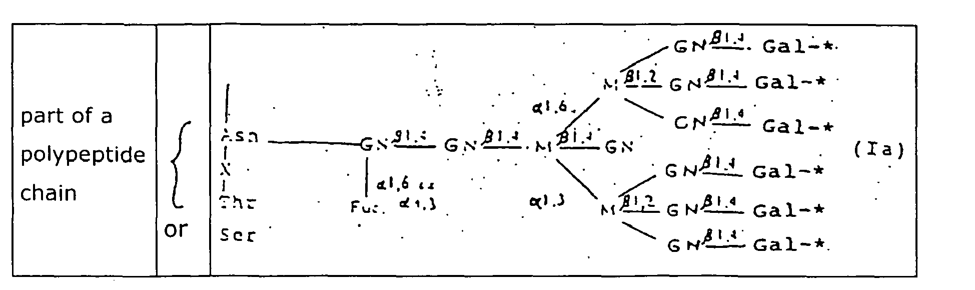 where:
Gal = galactose;
GN = N-acetyl-D-glucosamine;
M = D-mannose;
Fuc = fucose;
Asn = asparagine;
X = any amino acid with the exception of proline;
Thr = threonine;
Ser = serine;
* = attachment site of T (1 to 6 molecule radicals),
in which both peripheral radicals M can be substituted by 1 to 3 trisaccharides; or
T represents a saccharide radical having an O-glycan structure of formula (Ib): 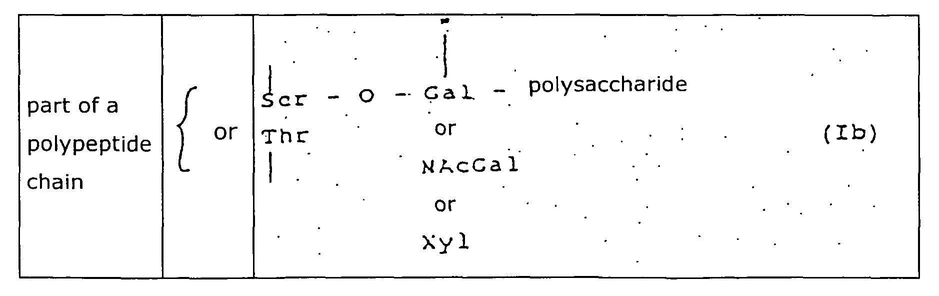 where:
Gal = galactose;
Thr ) threonine;
Ser = serine;
Xyl = xylose
NAcGal = N-acetylgalactosamine
* = attachment site of T
where in the above formulae (Ia) and (Ib) galactose (Gal) can be replaced by 2-deoxygalactose or 2-deoxy-2-halide(F, Cl, Br, I)-galactose.

4. The compounds of at least one of claims 1 to 3, wherein:
when T represents a saccharide radical having an N-glycan structure or represents a saccharide radical having an O-glycan structure, then GN represents a radical of general formula (II): 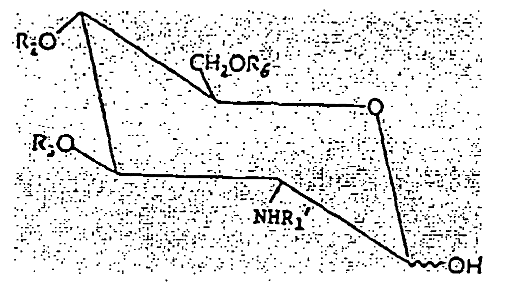
wherein R'₁, R₂ and R₃ have the meanings given above, R₆ has the same meanings as R₂ and R₃ and -NHR'₁ besides the equatorial position can also occupy an axial position, and wherein furthermore -OR₂ can occupy the axial position if -NHR'₁ takes an equatorial position, and wherein one or more or all of the groups -OR₂, -OR₃, -OR₆ and/or the free OH group can be acylated by a linear or branched, saturated or mono- or polyunsaturated acyl radical (-CO-R₁) having in total 1 to 20, preferably 1 to 7, carbon atoms, preferably by an acetyl radical that may have one or more hydroxy groups.

5. The compounds of at least one of claims 1 to 4, wherein R₁ to R₆ represent H or CH₃ or (C₁-C₇)-acyl, especially acetyl.

6. The compounds of at least one of claims 1 to 5, wherein said (C₃-C₇) acyl radical of R'₁ is a cyclopropanoyl radical that may have one or more hydroxy and/or carbonyl groups.

7. The compound of at least one of claims 1 to 6, wherein the recombinant human (rh) glycoprotein is rh differentiation factors, especially rh erythropoietin; rh growth factors, especially rh CSF (colony stimulating factor), rh GMCSF (granulocyte-monocyte stimulation factor); rh thrombolytic agents, especially rh tissue plasminogen activator (tPA) and rh urokinase; rh thromboprophylactic agents, especially rh antithrombin III; rh coagulation factors, especially rh factor VIII and rh factor IX; rh interferons, especially rh α-, β- and γ-interferons; and rh interleukins, especially rh IL-2, rh IL-15, rh IL-16 and rh IL-17.

8. The compounds of claim 7, which are recombinant human erythropoietin (rh erythropoietin) for the treatment of anemias in which the natural N-acetyl group of the glycan radicals, especially of the terminal N-acetylneuraminic acid radicals, is replaced by an N-(C₃-C₇)-acyl radical as defined in claim 6.

9. The compounds of claim 8, comprising one of the following glycan radicals: 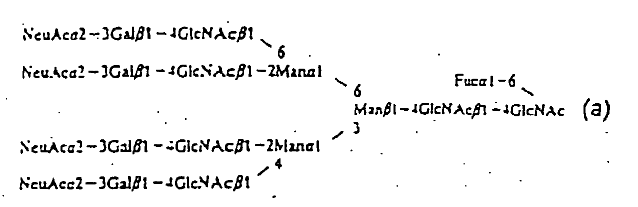 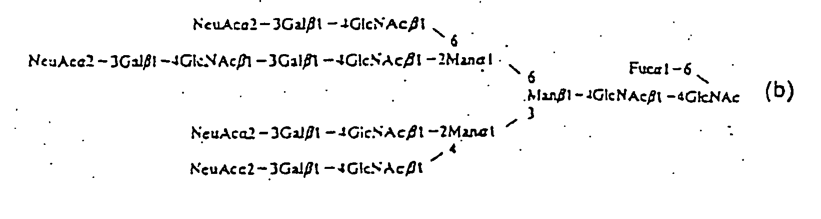 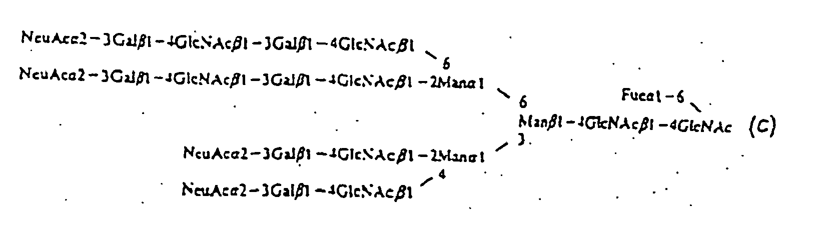 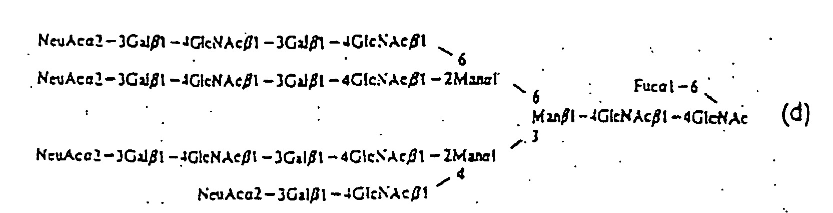 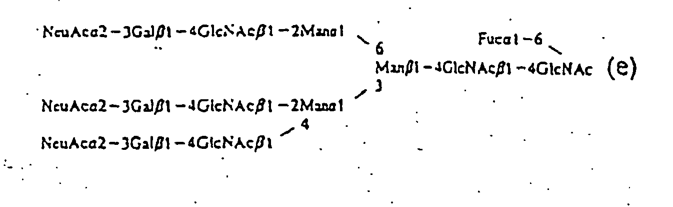 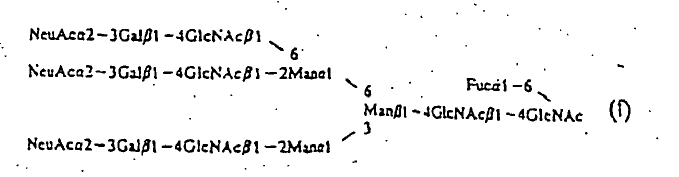 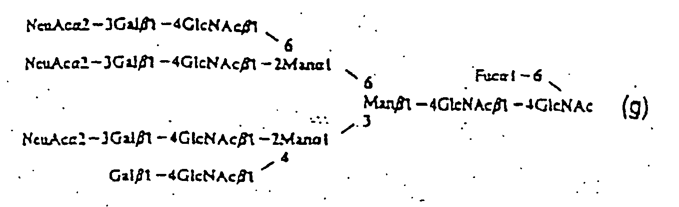 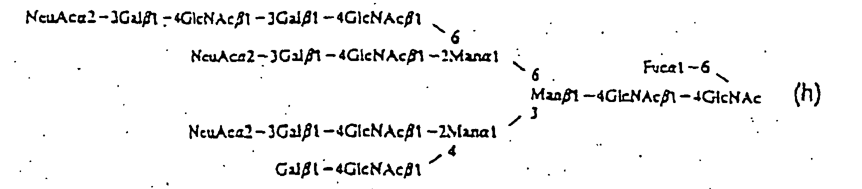 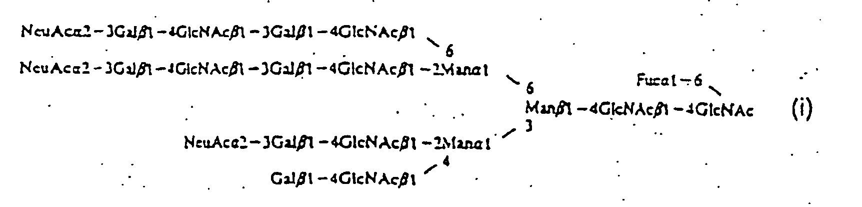 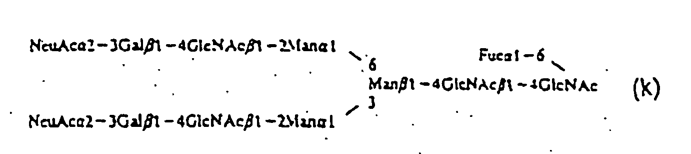 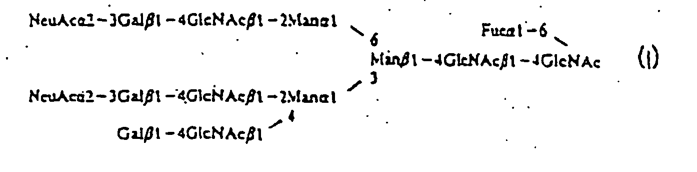 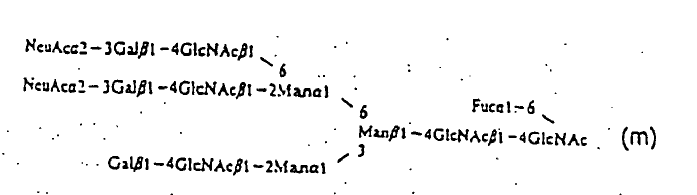 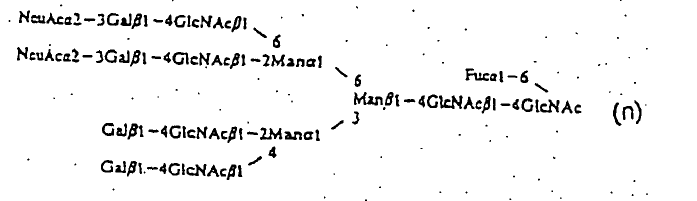 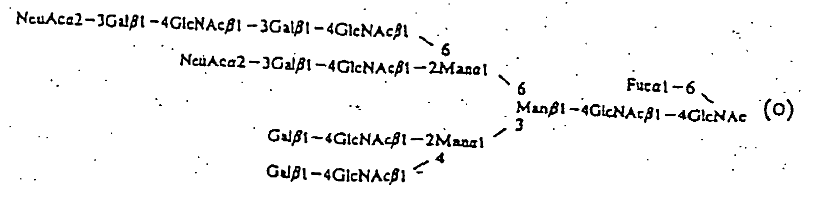 wherein Ac in the NeuAc radicals and/or one or more or all Ac in the remaining radicals respectively may be replaced by a (C₃-C₇) acyl radical as defined in claim 6.

10. The compounds of claim 7, which are rh growth factors, especially rh GCSF and/or rh GMCSF, for the treatment of diseases of the hematopoietic system, especially agranulocytosis or thrombocytopenia, mainly for the stimulation of the growth of white blood cells, especially lymphocytes, granulocytes and monocytes, in which the natural N-acetyl group of the glycan radicals, especially of the terminal N-acetylneuraminic acid radicals, is replaced by an N-(C₃-C₇)-acyl radical as defined in claim 6.

11. The compounds of claim 7, which are rh thrombolytic agents, especially rh tPA or rh urokinase, for the treatment of thrombi or for the prophylaxis of the formation of thrombi, in which the natural N-acetyl group of the glycan radicals, especially of the terminal N-acetylneuraminic acid radicals, is replaced by an N-(C₃-C₇)-acyl radical as defined in claim 6.

12. The compounds of claim 7, which are rh thromboprophylactic agents, especially rh antithrombin III, for the prophylaxis of the formation of thrombi, in which the natural N-acetyl group of the glycan radicals, especially of the terminal N-acetylneuraminic acid radicals, is replaced by an N-(C₃-C₇)-acyl radical as defined in claim 6.

13. The compounds of claim 7, which are rh coagulation factors, especially rh factor VIII and rh factor IX, for the treatment of disorders of the coagulation system due to an inherited or acquired deficiency of factor VIII and/or factor IX, in which the natural N-acetyl group of the glycan radicals, especially of the terminal N-acetylneuraminic acid radicals, is replaced by an N-(C₃-C₇)-acyl radical as defined in claim 6.

14. The compounds of claim 7, which are rh interferons, especially rh α-interferon compounds, for the treatment of malignant tumors, especially melanomas, and of autoimmune diseases, in which the natural N-acetyl group of the glycan radicals, especially of the terminal N-acetylneuraminic acid radicals, is replaced by an N-(C₃-C₇)-acyl radical as defined in claim 6.

15. The compounds of claim 7, which are rh interferons, especially rh β-interferon compounds, for the treatment of all forms of multiple sclerosis and viral diseases, mainly viral hepatitis B, viral hepatitis C, viral hepatitis D and viral encephalitis, in which the natural N-acetyl group of the glycan radicals, especially of the terminal N-acetylneuraminic acid radicals, is replaced by an N-(C₃-C₇)-acyl radical as defined in claim 6.

16. The compounds of claim 7, which are rh interferons, especially rh γ-interferon compounds, for the treatment of malignant tumors, viral hepatitis B, viral hepatitis C, viral hepatitis D and viral encephalitis, in which the natural N-acetyl group of the glycan radicals, especially of the terminal N-acetylneuraminic acid radicals, is replaced by an N-(C₃-C₇)-acyl radical as defined in claim 6.

17. The compounds of claim 7, which are rh interleukins, especially rh IL-2, rh IL-15, rh IL-16 and rh IL-17, for the treatment of malignant metastasing tumors, especially melanomas, in which the natural N-acetyl group of the glycan radicals, especially of the terminal N-acetylneuraminic acid radicals, is replaced by an N-(C₃-C₇)-acyl radical as defined in claim 6.

18. The compounds of at least one of claims 7 to 17, wherein the natural N-acetyl group of N-acetyl-D-glucosamine has been partly or completely replaced by N-cyclopropanoyl by a biochemical modulation (biochemical engineering) by the addition of the corresponding N-acyl-D-glucosamine or N-acyl-D-galactosamine or N-acyl-neuraminic acid, especially N-acyl-D-mannosamine, derivative to a culture medium in a final concentration in the medium of from 0.001 to 50.0 mM, especially from 0.5 to 20 mM, more specifically from 0.5 to 5 mM, where D-glucose in the said culture medium can be partly or completely replaced by D-galactose.

19. The compounds of at least one of claims 7 to 18, **characterized in that** the natural D-galactose has been partly or completely replaced by 2-deoxy-D-galactose by a biochemical modulation (biochemical engineering) by the addition of 2-deoxy-D-galactose to the culture medium in a final concentration in the medium of from 0.001 to 50 mM, especially from 0.5 to 20 mM, more specifically from 0.5 to 5 mM.

20. A process for producing the recombinant glycoproteins of at least one of claims 1 to 19 from a material containing one or more recombinant glycoproteins, especially recombinant human glycoproteins, and having been obtained from cell culture supernatants, wherein eukaryotic cells are cultivated, preferably in a culture medium containing as a glycan precursor an N-(C₃-C₇)-acylated monosaccharide for the biosynthesis of aminosugars in a concentration of from 0.001 to 50 mM, at a temperature of from 18 to 40 °C and at a pH of from 6.8 to 8.2, and the thus obtained N-acyl derivative of the glycoprotein is separated from the culture residue and recovered in a per se known manner, and optionally the OH groups or OR groups of the monosaccharide are mono- or poly- or completely acylated in a per se known manner.

21. The process of claim 20, wherein said eukaryotic cells are animal cells, preferably non-transformed CHO cells or COS7 cells or Nalm cells or fibroblasts.

22. The process of claim 20 or 21, wherein an FCS, RPMI1640, HAT, Eagle's MEM, Dulbecco's MEM and/or Glasgow MEM medium is (are) used as the culture medium.

23. The process of claim 20, 21 or 22, wherein N-isopropanoyl, -D-glucosamine, -D-galactosamine or -neuraminic acid, especially -D-mannosamine, or a mixture thereof is (are) used as a glycan precursor.

24. A pharmaceutical composition containing, as an active ingredient, at least one compound of one or more of claims 1 to 19, optionally in combination with other active ingredients and usual pharmaceutical vehicles and/or excipients.

25. The pharmaceutical composition of claim 24, **characterized by** containing, as an active ingredient, one or more recombinant human glycoproteins, or a mixture of differently modified N-(C₃-C₇)-acyl derivatives thereof.

26. The pharmaceutical composition of claim 24 or 25, containing the active ingredient or ingredients, which is (are) preferably mono- or polyacylated, preferably acetylated, at the monosaccharide, in an amount of from 0.001 to 50% by weight, preferably from 0.1 to 20% by weight, especially from 2 to 10% by weight.

27. The pharmaceutical composition of claims 24 to 26, containing, as an active ingredient, the compound of claims 7 to 9.

28. The pharmaceutical composition of claims 24 to 26, containing, as an active ingredient, the compound of claim 10.

29. The pharmaceutical composition of claims 24 to 26, containing, as an active ingredient, the compound of claim 11.

30. The pharmaceutical composition of claims 24 to 26, containing, as an active ingredient, the compound of claim 12.

31. The pharmaceutical composition of claims 24 to 26, containing, as an active ingredient, the compound of claim 13.

32. The pharmaceutical composition of claims 24 to 26, containing, as an active ingredient, the compound of claim 14.

33. The pharmaceutical composition of claims 24 to 26, containing, as an active ingredient, the compound of claim 15.

34. The pharmaceutical composition of claims 24 to 26, containing, as an active ingredient, the compound of claim 16.

35. The pharmaceutical composition of claims 24 to 26, containing, as an active ingredient, the compound of claim 17.

36. Use of the compounds of claims 8 and 9 for preparing a medicament for the treatment of anemias, especially renally caused anemias.

37. Use of the compounds of claim 10 for preparing a medicament for the treatment of diseases of the hematopoietic system, especially agranulocytosis or thrombocytopenia, mainly for the stimulation of the growth of white blood cells, especially lymphocytes, granulocytes and monocytes.

38. Use of the compounds of claim 11 for preparing a medicament for the treatment of thrombi or for the prophylaxis of the formation of thrombi.

39. Use of the compounds of claim 12 for preparing a medicament for the prophylaxis of the formation of thrombi.

40. Use of the compounds of claim 13 for preparing a medicament for the treatment of disorders of the coagulation system due to an inherited or acquired deficiency of factor VIII and/or factor IX.

41. Use of the compounds of claim 14 for preparing a medicament for the treatment of malignant tumors, especially melanomas, and of autoimmune diseases.

42. Use of the compounds of claim 15 for preparing a medicament for the treatment of all forms of multiple sclerosis and viral diseases, mainly viral hepatitis B, viral hepatitis C, viral hepatitis D and viral encephalitis.

43. Use of the compounds of claim 16 for preparing a medicament for the treatment of malignant tumors, viral hepatitis B, viral hepatitis C, viral hepatitis D and viral encephalitis.

44. Use of the compounds of claim 17 for preparing a medicament for the treatment of malignant metastasing tumors, especially melanomas.

45. Use of the compounds of claims 1 to 19 in their form which is mono- or polyacylated, preferably acetylated, at the OH or OR groups of the monosaccharide radical, for preparing a medicament for the purposes as stated in claims 36 to 44.

## Revendications

1. Glycoprotéines recombinantes de formule générale (I) 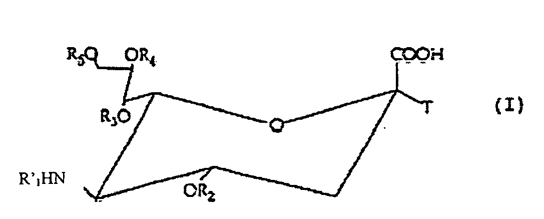 dans laquelle
R'₁ représente un radical acyle (en C₃ à C₇) cyclique, qui peut comporter un ou plusieurs groupe(s) hydroxyle et/ou carbonyle,
R₂, R₃, R₄ et R₅, qui peuvent être identiques ou différents, représentent respectivement l'hydrogène, un radical alkyle en C₁ à C₂₀, linéaire ou ramifié, un radical alcényle en C₃ à C₂₀, linéaire ou ramifié, un radical alcynyle en C₃ à C₂₀, linéaire ou ramifié, un radical alcényle ou alcynyle en C₄ à C₂₀, ayant 2 liaisons doubles ou triples, ou plus, un radical aryle en C₆ à C₂₀, un radical acyle (-CO-R₁), linéaire ou ramifié, saturé ou mono- ou polyinsaturé ayant un total de 1 à 20 atomes de carbone, qui peut comporter un ou plusieurs groupe(s) hydroxyle, un radical aroyle en C₆ à C₂₀, un radical carbonylamide de formule -CONH₂- ou - CONHR₁ ; un radical thioacyle (-CS-R₁), linéaire ou ramifié, saturé ou mono- ou polyinsaturé ayant un total de 1 à 20 atomes de carbone, ou un radical thiocarbamide de formule -CS-NH₂- ou -CS-NHR₁, dans laquelle R₁ représente respectivement l'hydrogène ou un radical alkyle en C₁ à C₂₀, linéaire ou ramifié, saturé ou mono- ou polyinsaturé, et où chacun des radicaux sus-mentionnés à l'exception de H peut comprendre en tant que substituant(s) un ou plusieurs groupe(s) halogéno, hydroxy, époxy, amino, mercaptan, phényle, phénol ou benzyle, et
T représente un radical mono-, di- ou oligosaccharide comportant jusqu'à 40 radicaux sucre liés entre eux par liaison glycosidique, qui représentent des cycles furanose et/ou pyranose et qui comprennent 5 à 230 atomes de carbone et qui sont liés à des polypeptides par une liaison N- ou O-glycosidique.

2. Glycoprotéines selon la revendication 1, où, dans la formule générale (I) :
R₂, R₃, R₄ et R₅, qui peuvent être identiques ou différents, représentent respectivement l'hydrogène, un radical alkyle linéaire ou ramifié ayant 1 à 7 atomes de carbone, un radical alcényle linéaire ou ramifié ayant 3 à 10 atomes de carbone, un radical alcynyle linéaire ou ramifié ayant 3 à 10 atomes de carbone, un radical alcényle ou alcynyle ayant 2 liaisons doubles ou triples, ou plus, ayant 7 à 12 atomes de carbone, un radical phényle, un radical acyle (-COR₁), linéaire ou ramifié, saturé ou mono- ou polyinsaturé ayant un total de 1 à 7 atomes de carbone, en particulier un radical acétyle pouvant comporter un ou plusieurs groupe(s) hydroxyle, un radical aroyle ayant 6 à 10 atomes de carbone, un radical carbonylamide de formule -CONH₂- ou -CONHR₁, un radical thioacyle (-CS-R₁), linéaire ou ramifié, saturé ou mono- ou polyinsaturé ayant un total de 1 à 7 atomes de carbone, ou un radical thiocarbamide de formule -CS-NH₂- ou -CS-NHR₁, où R₁ représente respectivement l'hydrogène ou un radical alkyle linéaire ou ramifié, saturé ou mono- ou polyinsaturé, ayant 1 à 20 atomes de carbone, et où chacun des radicaux sus-mentionnés à l'exception de H peut être éventuellement mono- ou polysubstitué par des groupes fluor, chlore, brome ou iode, hydroxy, époxy, amino, mercaptan, phényle, phénol ou benzyle.

3. Composés selon la revendication 1 ou 2, où dans la formule générale (I), T représente un radical saccharide avec une structure N-glycane de formule (Ia) : 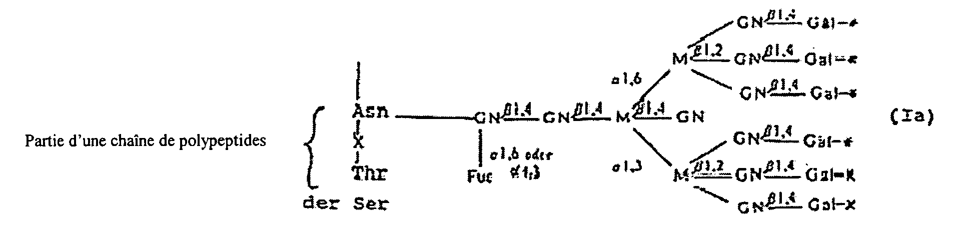 dans laquelle :
Gal = galactose,
GN = N-acétyl-D-glucosamine,
M = D-mannose,
Fuc = fucose,
Asn = asparagine,
X = un acide aminé quelconque hormis proline,
Thr = thréonine,
Ser = sérine,
* = site de liaison à T (1 à 6 radicaux moléculaires),
où les deux radicaux périphériques M peuvent être substitués par 1 à 3 trisaccharide(s) ;
ou bien représente un radical saccharide de structure O-glycane de formule générale (Ib) : 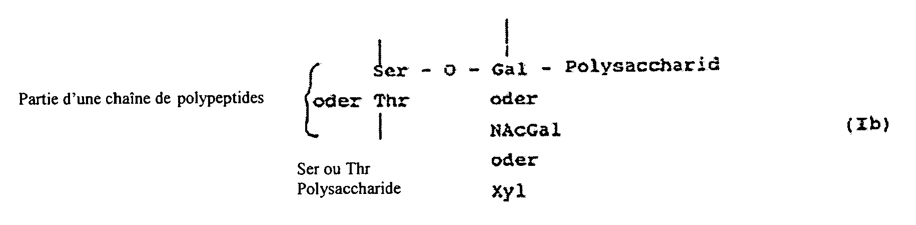 dans laquelle :
Gal = galactose,
Thr = thréonine,
Ser = sérine,
Xyl = xylose,
NacGal = N-acétyl-galactosamine,
* = site de liaison à T,
où, dans les formules (Ia) et Ib) ci-dessus, le galactose (Gal) peut être substitué par le 2-désoxygalactose ou le 2-désoxy-2-halogénure(F, Cl, Br, I)-galactose.

4. Composés selon au moins l'une des revendications 1 à 3, où dès lors que T est un radical saccharide de structure N-glycane ou un radical saccharide de structure O-glycane, alors GN représente un radical de formule générale (II) : 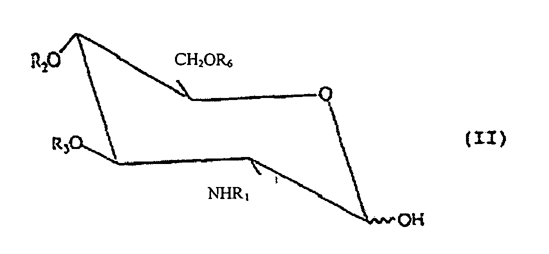 dans laquelle
R'₁, R₂, R₃ sont tels que définis précédemment, R₆ a les mêmes significations que R₂ et R₃ et NHR'₁- peut, outre la position équatoriale, également adopter la position axiale, et dans laquelle, en outre, en cas de position équatoriale de -NHR'₁-, -OR₂ peut se trouver en position axiale, et dans laquelle un ou plusieurs ou tous les groupes -OR₂, -OR₃, -OR₆ et/ou le groupe OH libre peut ou peuvent être acylé(s) par un radical acyle (-CO-R₁) linéaire ou ramifié, saturé ou mono- ou polyinsaturé, ayant un total de 1 à 20, de préférence de 1 à 7 atomes de carbone, de préférence par le radical acétyle pouvant comporter un ou plusieurs groupe(s) hydroxyle.

5. Composés selon au moins l'une des revendications 1 à 4, où R₁ à R₆ représentent H ou CH₃ ou un radical acyle en C₁ à C₇, en particulier un radical acétyle.

6. Composés selon au moins l'une des revendications 1 à 5, où le radical acyle en C₃ à C₇ de R'₁ est un radical cyclopropanoyle, qui peut comporter un ou plusieurs groupe(s) hydroxyle et/ou carbonyle.

7. Composé selon au moins l'une des revendications 1 à 6, où il s'agit dans le cas de la glycoprotéine rh de facteurs de différenciation rh, en particulier de l'érythropoïétine rh ; de facteurs de croissance rh, en particulier du facteur CSF rh (facteur de stimulation des colonies), du facteur de croissance hématopoïétique GM-CSF rh (facteur de stimulation des granulocytes-monocytes); de thrombolytiques rh, en particulier de l'activateur tissulaire du plasminogène (tPA) rh et de l'urokinase rh ; de thromboprophylactiques rh, en particulier de l'antithrombine III rh ; de facteurs de coagulation rh, en particulier du facteur VIII rh et du facteur IX rh ; d'interférons rh, en particulier des interférons alpha, bêta et gamma rh ; et d'interleukines rh, en particulier des interleukines IL-2 rh, IL-15 rh, IL-16 rh et IL-17 rh.

8. Composés selon la revendication 7, où il s'agit ici d'érythropoïétine recombinante humaine (érythropoïétine rh) pour le traitement des anémies, dans laquelle le groupe N-acétyle naturel des radicaux glycane, en particulier des radicaux acide N-acétylneuraminique terminaux, est substitué par un radical N-(C₃-C₇) acyle tel que défini dans la revendication 6.

9. Composés selon la revendication 8, qui comportent l'un des radicaux glycane suivants : 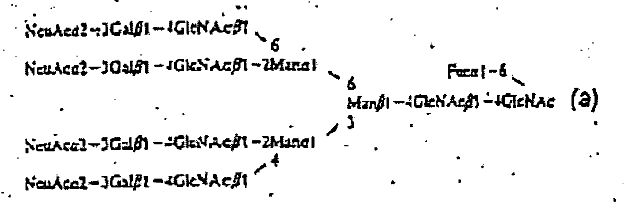 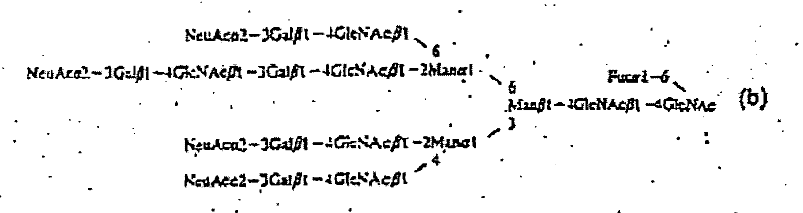 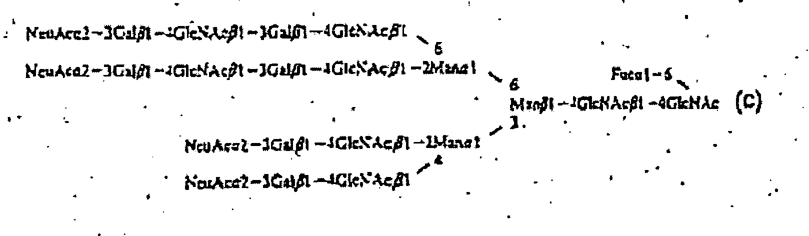 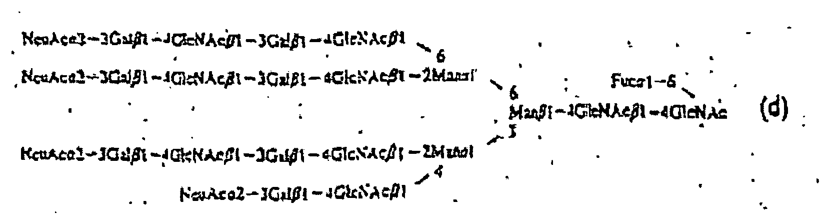 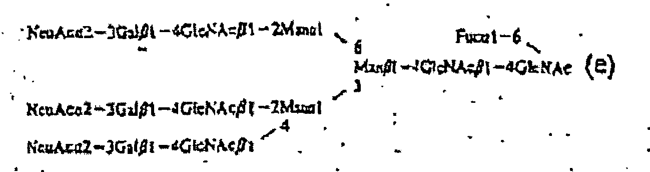 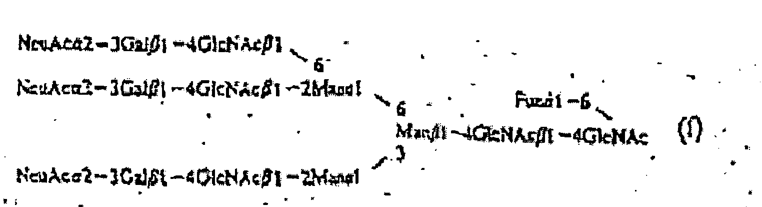 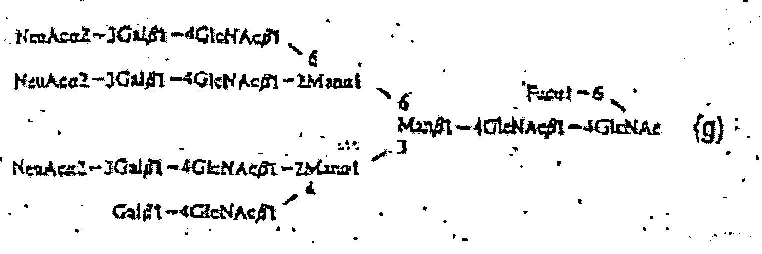 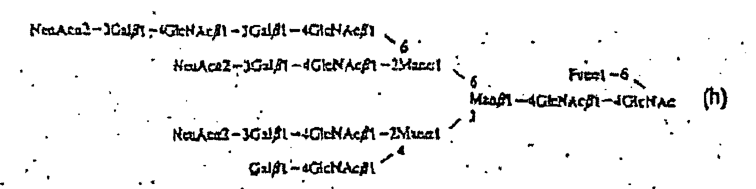 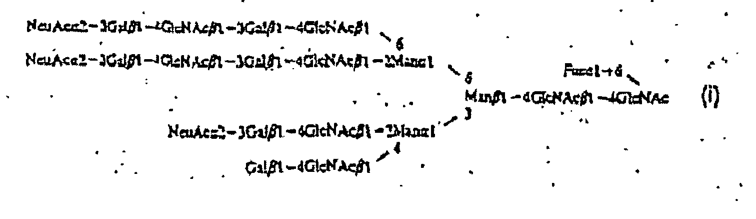 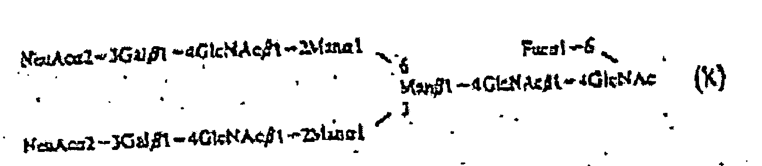 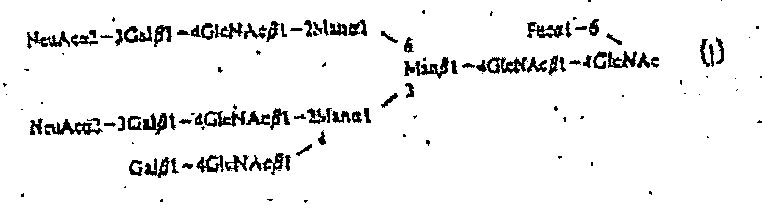 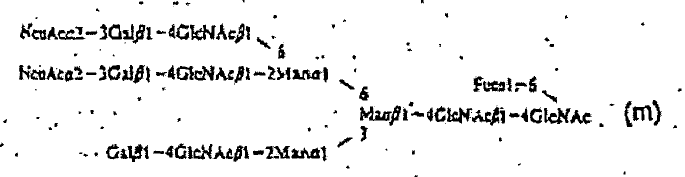 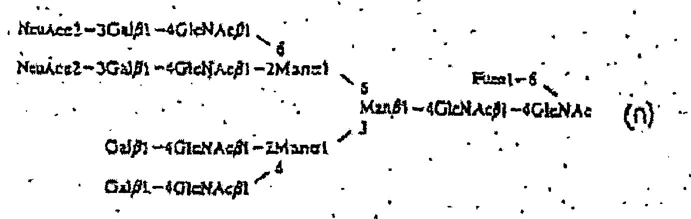 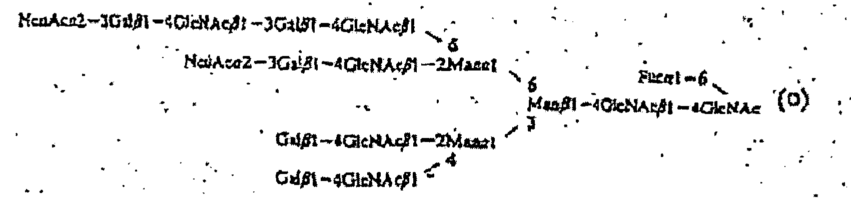 où Ac dans les radicaux NeuAc et/ou un ou plusieurs parmi ou tous les Ac dans les autres radicaux est ou sont chaque fois substitué(s) par un radical acyle en (C₃-C₇) tel que défini dans la revendication 6.

10. Composés selon la revendication 7, où il s'agit de facteurs de croissance rh, en particulier du facteur de croissance G-CSF rh et/ou du facteur de croissance GM-CSF rh, pour le traitement de maladies du système produisant les cellules sanguines, en particulier de l'agranulocytose ou de la thrombopénie, avant tout pour stimuler la croissance des globules blancs, en particulier des lymphocytes, des granulocytes et des monocytes, dans lesquels le groupe N-acétyle naturel des radicaux glycane, en particulier des radicaux acide N-acétylneuraminique terminaux, est substitué par un radical N-(C₃-C₇)-acyle tel que défini dans la revendication 6.

11. Composés selon la Revendication 7, où il s'agit de thrombolytiques rh, en particulier du tPA rh ou de l'urokinase rh, pour le traitement des thrombus ou pour prévenir la formation de thrombus, dans lesquels le groupe N-acétyle naturel des radicaux glycane, en particulier des radicaux acide N-acétylneuraminique terminaux, est substitué par un radical N-(C₃-C₇)-acyle tel que défini dans la revendication 6.

12. Composés selon la revendication 7, où il s'agit de thromboprophylactiques rh, en particulier de l'antithrombine III rh, pour prévenir la formation de thrombus, dans lesquels le groupe N-acétyle naturel des radicaux glycane, en particulier des radicaux acide N-acétylneuraminique terminaux, est substitué par un radical N-(C₃-C₇)-acyle tel que défini dans la revendication 6.

13. Composés selon la revendication 7, où il s'agit de facteurs de coagulation recombinants humains, en particulier du facteur VIII rh et/ou du facteur IX rh, pour le traitement de troubles du système de la coagulation provoqués par une carence acquise ou héréditaire en facteur VIII et/ou en facteur IX, dans lesquels le groupe N-acétyle naturel des radicaux glycane, en particulier des radicaux acide N-acétylneuraminique terminaux, est substitué par un radical N-(C₃-C₇)-acyle tel que défini dans la revendication 6.

14. Composés selon la revendication 7, où il s'agit d'interférons rh, en particulier de composés d'interféron alpha rh, pour le traitement de tumeurs malignes, en particulier de mélanomes, et de maladies d'autoagression, dans lesquels le groupe N-acétyle naturel des radicaux glycane, en particulier des radicaux acide N-acétylneuraminique terminaux, est substitué par un radical N-(C₃-C₇)-acyle tel que défini dans la revendication 6.

15. Composés selon la revendication 7, où il s'agit d'interférons rh, en particulier de composés d'interféron bêta recombinant humain, pour le traitement de toutes les formes de sclérose multiple et de maladies virales, avant tout du virus de l'hépatite B, du virus de l'hépatite C, du virus de l'hépatite D et des encéphalites virales, dans lesquels le groupe N-acétyle naturel des radicaux glycane, en particulier des radicaux acide N-acétylneuramique terminaux, est substitué par un radical N-(C₃-C₇)-acyle tel que défini dans la revendication 6.

16. Composés selon la revendication 7, où il s'agit d'interférons rh, en particulier de composés d'interféron gamma rh, pour le traitement de tumeurs malignes, du virus de l'hépatite B, du virus de l'hépatite C, du virus de hépatite D et des encéphalites virales, dans lesquels le groupe N-acétyle naturel des radicaux glycane, en particulier des radicaux acide N-acétylneuraminique terminaux, est substitué par un radical N-(C₃-C₇)-acyle tel que défini dans la revendication 6.

17. Composés selon la revendication 7, où il s'agit d'interleukines rh, en particulier des interleukines IL-2 rh, IL-15 rh, IL-16 rh et IL-17 rh, pour le traitement de tumeurs métastatiques malignes, en particulier de mélanomes, dans lesquels le groupe N-acétyle naturel des radicaux glycane, en particulier des radicaux acide N-acétylneuraminique terminaux, est substitué par un radical N-(C₃-C₇)-acyle tel que défini dans la revendication 6.

18. Composés selon au moins l'une des revendications 7 à 17, dans lesquels le groupe N-acétyle naturel de la N-acétyl-D-glucosamine a été substitué totalement ou partiellement par un N-cyclopropanoyle par modulation biochimique (Biochemical-Engineering) par adjonction au milieu de culture respectivement correspondant du dérivé de N-acyl-D-glucosamine ou de N-acyl-D-galactosamine ou d'acide N-acyl-neuraminique, en particulier de N-acyl-D-mannosamine, à une concentration finale dans le milieu de 0,001 à 50,0 mM, en particulier de 0,5 à 20 mM, particulièrement de 0,5 à 5 mM, le D-glucose dans le milieu de culture mentionné pouvant être totalement ou partiellement substitué par le D-galactose.

19. Composés selon au moins l'une des revendications 7 à 18, **caractérisé en ce que** le D-galactose naturel a été totalement ou partiellement substitué par du 2-désoxy-D-galactose par modulation biochimique (Biochemical-Engineering) par adjonction au milieu de culture de 2-désoxy-D-galactose, à une concentration finale dans le milieu de 0,001 à 50,0 mM, en particulier de 0,5 à 20 mM, tout particulièrement de 0,5 à 5 mM.

20. Procédé de préparation de glycoprotéines recombinantes selon au moins l'une des revendications 1 à 19, à partir d'un matériau contenant une ou plusieurs glycoprotéine(s) recombinante(s), en particulier des glycoprotéines humaines recombinantes, que l'on a recueilli à partir de surnageants de cultures cellulaires, où des cellules eucaryotes sont cultivées, de préférence dans un milieu de culture contenant en tant que précurseur de glycane un N-(C₃-C₇)-monosaccharide acylé pour la biosynthèse des aminosucres à une concentration de 0,001 à 50 mMol/l, à une température de 18 à 40 °C et à un pH de 6,8 à 8,2, et le dérivé N-acyle de glycoprotéine ainsi obtenu est séparé du reste de la culture de façon connue en soi, puis récupéré, et éventuellement acylé de façon connue en soi de façon simple, multiple ou totale aux groupes OH- ou OR- du monosaccharide.

21. Procédé selon la revendication 20, dans lequel les cellules eucaryotes sont des cellules animales, de préférence des cellules CHO ou des cellules COS7 ou encore des cellules Nalm ou des fibroblastes, non transformé(e)s.

22. Procédé selon la revendication 20 ou 21, dans lequel on utilise en tant que milieu de culture un milieu FCS, RPMI 1640, HAT, Eagle-MEM, Dulbecco-MEM et/ou un milieu Glasgow-MEM.

23. Procédé selon la revendication 20, 21 ou 22, dans lequel on utilise en tant que précurseur de glycane du N-isopropanoyle, de la D-glucosamine, de la D-galactosamine ou de l'acide neuraminique, en particulier de la D-mannosamine, ou un de leurs mélanges.

24. Produit pharmaceutique contenant en tant que principe actif au moins un composé selon une ou plusieurs des revendications 1 à 19, éventuellement en combinaison avec d'autres principes actifs, ainsi qu'avec les excipients et/ou les auxiliaires pharmaceutiques usuels.

25. Produit pharmaceutique selon la revendication 24, **caractérisé en ce qu'**il contient en tant que principe actif une ou plusieurs glycoprotéines recombinantes humaines, ou un mélange de dérivés N-(C₃-C₇)-acyle diversement modifiés.

26. Produit pharmaceutique selon la revendication 24 ou 25, contenant le ou les principe(s) actif(s) de préférence acylé(s) de façon simple ou multiple au niveau du monosaccharide, de préférence acétylé(s) en quantité de 0,001 à 50 % en poids, de préférence de 0,1 à 20 % en poids, en particulier de 2 à 10 % en poids.

27. Produit pharmaceutique selon les revendications 24 à 26, contenant en tant que principe actif le composé selon les revendications 7 à 9.

28. Produit pharmaceutique selon les revendications 24 à 26, contenant en tant que principe actif le composé selon la revendication 10.

29. Produit pharmaceutique selon les revendications 24 à 26, contenant en tant que principe actif le composé selon la revendication 11.

30. Produit pharmaceutique selon les revendications 24 à 26, contenant en tant que principe actif le composé selon la revendication 12.

31. Produit pharmaceutique selon les revendications 24 à 26, contenant en tant que principe actif le composé selon la revendication 13.

32. Produit pharmaceutique selon les revendications 24 à 26, contenant en tant que principe actif le composé selon la revendication 14.

33. Produit pharmaceutique selon les revendications 24 à 26, contenant en tant que principe actif le composé selon la revendication 15.

34. Produit pharmaceutique selon les revendications 24 à 26, contenant en tant que principe actif le composé selon la revendication 16.

35. Produit pharmaceutique selon les revendications 24 à 26, contenant en tant que principe actif les composés selon la revendication 17.

36. Utilisation des composés selon les revendications 8 et 9, pour la fabrication d'un médicament destiné au traitement des anémies, en particulier des anémies provoquées au niveau rénal.

37. Utilisation des composés selon la revendication 10, pour la fabrication d'un médicament destiné au traitement de maladies du système produisant les cellules sanguines, en particulier de l'agranulocytose ou de la thrombopénie, avant tout pour stimuler la croissance des globules blancs, en particulier des lymphocytes, des granulocytes et des monocytes.

38. Utilisation des composés selon la revendication 11, pour la fabrication d'un médicament destiné au traitement des thrombus ou à prévenir la formation de thrombus.

39. Utilisation des composés selon la revendication 12, pour la fabrication d'un médicament destiné à prévenir la formation des thrombus.

40. Utilisation des composés selon la revendication 13, pour la fabrication d'un médicament destiné au traitement de troubles du système de la coagulation provoqués par une carence acquise ou héréditaire en facteur VIII et/ou en facteur IX.

41. Utilisation des composés selon la revendication 14, pour la fabrication d'un médicament destiné au traitement de tumeurs malignes, en particulier de mélanomes et de maladies d'autoagression.

42. Utilisation des composés selon la revendication 15, pour la fabrication d'un médicament destiné au traitement de toutes les formes de scléroses multiples et de maladies virales, avant tout du virus de l'hépatite B, du virus de l'hépatite C, du virus de hépatite D et des encéphalites virales.

43. Utilisation des composés selon la revendication 16, pour la fabrication d'un médicament destiné au traitement de tumeurs malignes, du virus de l'hépatite B, du virus de l'hépatite C, du virus de hépatite D et des encéphalites virales.

44. Utilisation des composés selon la revendication 17, pour la fabrication d'un médicament destiné au traitement de tumeurs métastatiques malignes, en particulier de mélanomes.

45. Utilisation des composés selon les revendications 1 à 19, sous leur forme acylée, de préférence acétylée, de façon simple ou multiple aux groupes OH- ou OR- du radical monosaccharide, pour la fabrication d'un médicament destiné aux buts mentionnés dans les revendications 36 à 44.
